# EUROPÄISCHE PATENTSCHRIFT

(11) **EP 3 505 237 B1**
(45) Veröffentlichungstag und Bekanntmachung des Hinweises auf die Patenterteilung: **29.07.2020**
(21) Anmeldenummer: 18207305.6
(22) Anmeldetag: 20.11.2018
(51) Int. Cl.: B01F 15/02

(54) **KNOCHENZEMENTMISCHVORRICHTUNG UND VERFAHREN MIT ABSTANDHALTER IN EINER AMPULLENAUFNAHME**
BONE CEMENT MIXING APPARATUS AND METHOD WITH SPACER IN AN AMPOULE HOLDER
DISPOSITIF ET PROCEDE DE MÉLANGE DE CIMENT OSSEUX POURVU D'ÉCARTEUR DANS UN LOGEMENT À AMPOULE

(30) Priorität: 15.12.2017 DE 102017130084
(43) Veröffentlichungstag der Anmeldung: 03.07.2019
(73) Patentinhaber: Heraeus Medical GmbH, 61273 Wehrheim (DE)
(72) Erfinder: Vogt, Sebastian, 99092 Erfurt (DE); Kluge, Thomas, 56179 Vallendar (DE); Strathausen, Rainer, 61381 Friedrichsdorf (DE)
(74) Vertreter: Heraeus IP

(56) Entgegenhaltungen:
- DE-A1- 4 030 832

## Beschreibung

Die Erfindung betrifft eine Vorrichtung zum Herstellen eines Knochenzementteigs aus einer Monomerflüssigkeit und einem Zementpulver als Ausgangskomponenten des Knochenzementteigs und zum Austragen des gemischten Knochenzementteigs.

Die Erfindung betrifft auch ein Verfahren zur Herstellung eines Knochenzementteigs, insbesondere eines pastenförmigen Polymethylmethacrylat-Knochenzementteigs.

Gegenstand der Erfindung ist insbesondere eine Vorrichtung zum Lagern, Vermischen und Applizieren von Polymethylmethacrylat-Knochenzement. Die Vorrichtung ist für die Zementierung von Totalgelenkendoprothesen bestimmt. Es handelt sich bei der erfindungsgemäßen Vorrichtung bevorzugt um ein Full-Prepacked-Zementiersystem.

Polymethylmethacrylat-(PMMA)-Knochenzemente gehen auf die grundlegenden Arbeiten von Sir Charnley zurück (Charnley, J.: Anchorage of the femoral head prosthesis of the shaft of the femur. J. Bone Joint Surg. 42 (1960) 28-30). Konventionelle Polymethylmethacrylat-Knochenzemente (PMMA-Knochenzemente) sind aus einer pulverförmigen Komponente und einer flüssigen Monomerkomponente zusammengesetzt (K.-D. Kühn: Knochenzemente für die Endoprothetik: Ein aktueller Vergleich der physikalischen und chemischen Eigenschaften handelsüblicher PMMA-Zemente. Springer-Verlag Berlin Heidelberg New York, 2001). Die Monomerkomponente enthält im Allgemeinen das Monomer Methylmethacrylat und einen darin gelösten Aktivator (N,N-Dimethyl-p-toluidin). Die Pulverkomponente, auch als Zementpulver oder Knochenzementpulver bezeichnet, weist ein oder mehrere Polymere auf, die auf Basis von Methylmethacrylat und Comonomeren, wie Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement beziehungsweise Knochenzementteig. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Knochenzementteigs, bis dieser erstarrt.

PMMA-Knochenzemente können in geeigneten Mischbechern mit Hilfe von Spateln durch Vermischen des Zementpulvers mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können.

Zur Vermeidung von Lufteinschlüssen im Knochenzementteig wurden eine Vielzahl von Vakuum-Zementiersystemen beschrieben, von denen exemplarisch folgende genannt sind: US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, EP 1 005 901 A2, EP 1 886 647 A1, US 5 344 232 A.

Eine Weiterentwicklung in der Zementiertechnik stellen Zementiersysteme dar, in denen sowohl das Zementpulver als auch die Monomerflüssigkeit bereits in separaten Kompartimenten der Mischvorrichtungen verpackt sind und die erst unmittelbar vor der Zementapplikation im Zementiersystem miteinander vermischt werden. Solche geschlossenen Full-Prepacked-Mischvorrichtungen wurden mit der EP 0 692 229 A1, der DE 10 2009 031 178 B3, der US 5 997 544 A, der US 6 709 149 B1, der WO 00/35506 A1, der EP 0 796 653 A2 und der US 5 588 745 A vorgeschlagen.

Das Patent DE 10 2009 031 178 B3 offenbart eine Lager- und Mischvorrichtung als Full-Prepacked-Mischvorrichtung, in dem die zur Herstellung des Knochenzementteigs notwendigen Ausgangskomponenten bereits in der Lager- und Mischvorrichtung gelagert sind und in der Lager- und Mischvorrichtung zusammengeführt und gemischt werden können. Die Lager- und Mischvorrichtung weist einen zweiteiligen Austragskolben zum Verschluss einer Zementkartusche auf. Dabei wird eine Kombination aus einem gasdurchlässigen Sterilisationskolben und einem gasundurchlässigen Dichtungskolben verwendet.

Die Patentanmeldung DE 40 30 832 A1 offenbart ein Full-Prepacked-Zementiersystem, bei dem Monomerflüssigkeit nach Öffnung einer Ampulle in einen Pulverbehälter einer Kartusche enthaltend ein Zementpulver gezogen wird. Die Vermischung des Zementpulvers mit der Monomerflüssigkeit erfolgt durch Schütteln der Kartusche. Das Öffnen der Ampulle wird durch axiales Einschieben eines von außen zugänglichen Kolbens in die Kartusche bewirkt. Der Kolben schiebt die Ampulle gegen eine Halterung und bricht dadurch einen Ampullenkopf, der in der Halterung gelagert ist, von der Ampulle ab, um die Monomerflüssigkeit aus der Ampulle freizusetzen.

Polymethylmethacrylat-Knochenzemente werden nach Vermischung des Zementpulvers mit der flüssigen Monomerkomponente im noch nicht ausgehärteten, pastenförmigen Zustand als Knochenzementteig appliziert. Bei Verwendung von Mischvorrichtungen befindet sich der Knochenzementteig im Fall von Pulver-Flüssigkeits-Zementen in einer Kartusche. Bei der Applikation solcher konventioneller PMMA-Knochenzemente, wird nach der Vermischung der beiden Ausgangskomponenten der gebildete Knochenzementteig mit Hilfe von manuell bedienbaren Auspressvorrichtungen ausgepresst. Aus der Kartusche wird der Knochenzementteig durch Bewegung eines Austragskolbens herausgedrückt. Die Austragskolben haben üblicherweise einen Durchmesser zwischen 30 mm bis 40 mm und damit an der Außenseite, an welcher ein Stößel beziehungsweise eine Stange der Auspressvorrichtung beim Auspressvorgang angreift, eine Fläche von 7,0 cm² bis 12,5 cm². Die Bewegung des Austragskolbens wird bevorzugt durch manuell bedienbare, mechanische Auspressvorrichtungen bewirkt. Diese manuellen Auspressvorrichtungen erreichen normalerweise eine Auspresskraft im Bereich von ungefähr 1,5 kN bis 3,5 N.

Diese einfachen mechanischen Auspressvorrichtungen nutzen insbesondere Klemmstangen zum Auspressen, die durch einen manuell zu betätigenden Kipphebel angetrieben werden. Die manuell angetriebenen Auspressvorrichtungen sind seit Jahrzehnten weltweit erprobt und stellen bisher den Stand der Technik dar. Vorteilhaft an diesen Auspressvorrichtungen ist, dass der medizinische Anwender über die aufzubringende Handkraft ein Gefühl für den Eindringwiderstand des Knochenzementteigs in die Knochenstrukturen (Spongiosa) hat.

Bei der Verwendung aller bisher bekannten Full-Prepacked-Mischvorrichtungen muss der medizinische Anwender mehrere Arbeitsschritte in einer vorbestimmten Reihenfolge an den Vorrichtungen nacheinander durchführen bis der gemischte Knochenzementteig vorliegt und appliziert werden kann. Eine Verwechslung der Arbeitsschritte kann zum Versagen der Mischvorrichtung führen und daher Störungen im OP-Ablauf verursachen. Kostenintensive Schulungen der medizinischen Anwender sind deshalb notwendig, um Anwenderfehler zu vermeiden.

In der WO 00/35506 A1 wird eine Vorrichtung vorgeschlagen, bei der Polymethylmethacrylat-Zementpulver in einer Kartusche gelagert wird, wobei das Zementpulver das gesamte Volumen der Kartusche ausfüllt und die Zwischenräume zwischen den Partikeln des Zementpulvers ein solches Volumen hat, das dem Volumen der Monomerflüssigkeit entspricht, das zur Herstellung von Knochenzementteig mit dem in der Kartusche gelagerten Zementpulver notwendig ist. Diese Vorrichtung ist so aufgebaut, dass durch Vakuumeinwirkung die Monomerflüssigkeit von oben in die Kartusche eingeleitet wird, wobei hierzu ein Vakuum an einem Vakuumanschluss an der Unterseite der Kartusche angelegt wird. Dadurch wird die Monomerflüssigkeit durch das Zementpulver gezogen, wobei die in den Zwischenräumen der Zementpulverpartikel befindliche Luft durch die Monomerflüssigkeit verdrängt wird. Auf eine mechanische Durchmischung des gebildeten Zementteigs mit einem Rührer wird dabei verzichtet.

Nachteilig an diesem System ist, dass Zementpulver, die schnell mit der Monomerflüssigkeit anquellen, mit dieser Vorrichtung nicht angemischt werden können, weil die schnell anquellenden Zementpulverpartikel nach einem Eindringen der Monomerflüssigkeit in das Zementpulver von ungefähr 1 bis 2 cm eine gelartige Barriere bilden und die Migration der Monomerflüssigkeit durch das gesamte Zementpulver behindern. Konventionelle Zementpulver zeigen zudem das Phänomen, dass auf Grund der unterschiedlichen Oberflächenenergien die Zementpulverpartikel durch Methylmethacrylat nur schlecht benetzt werden. Dadurch dringt das Methylmethacrylat nur relativ langsam in das Zementpulver ein. Weiterhin kann bei Vakuumeinwirkung nicht ausgeschlossen werden, dass nach vollständiger Durchdringung des Zementpulvers durch die Monomerflüssigkeit die Monomerflüssigkeit über den Vakuumanschluss abgesaugt wird. Dann steht nicht genügend Monomerflüssigkeit für die Aushärtung durch radikalische Polymerisation zur Verfügung beziehungsweise das Mischungsverhältnis wird ungewollt verändert und damit auch die Konsistenz des Knochenzementteigs. Weiterhin ist es problematisch, dass die zwischen den Zementpulverpartikeln eingeschlossen Luft durch die Monomerflüssigkeit von oben nach unten verdrängt werden soll, weil die gegenüber der Monomerflüssigkeit spezifisch leichtere Luft auf Grund der Schwerkraft das Bestreben hat, im Zementpulver nach oben zu wandern und nicht nach unten in Richtung Vakuumanschluss zu migrieren.

In der DE 10 2016 121 607 wurde ein Full-Prepacked-Mischsystem mit einer Kartusche enthaltend ein Zementpulver zur Herstellung eines Knochenzementteigs vorgeschlagen. In der Kartusche ist ein Austragskolben vorgesehen und hinter der Kartusche eine Aufnahme enthaltend einen Monomerflüssigkeitsbehälter angeordnet. An der Rückseite der Aufnahme befindet sich ein Förderkolben, mit dem der Monomerflüssigkeitsbehälter zerquetscht werden kann und die Monomerflüssigkeit aus der Aufnahme in die Kartusche gepresst werden kann.

Es zeigte sich in praktischen Versuchen, dass der mit dieser Vorrichtung hergestellte Knochenzementteig bei der Verwendung eines geeigneten Zementpulvers immer eine gute Konsistenz besitzt. Wird der geborstene Monomerflüssigkeitsbehälter beim Monomertransfer maximal komprimiert, dann erhält man reproduzierbar einen guten Zementteig. Bei unvollständiger Kompression des geborstenen Monomerflüssigkeitsbehälters kann jedoch ein Monomerflüssigkeitsrest zwischen dem Austragskolben und dem Förderkolben innerhalb der Fragmente des geborstenen Monomerflüssigkeitsbehälters verbleiben, der am Ende des Auspressens des Zementteigs durch eine anschließende Nachkompression des geborstenen Monomerflüssigkeitsbehälters infolge einer axialen Bewegung des Förderkolbens in Richtung des Austragskolbens austreten kann. Dieser Monomerflüssigkeitsrest kann die Konsistenz des Knochenzementteigs während das Austragens verändern. Auch können unerwünschte Monomerblasen in dem Knochenzementteig entstehen.

Die Aufgabe der vorliegenden Erfindung besteht also darin, die Nachteile des Stands der Technik zu überwinden. Insbesondere besteht die Aufgabe der Erfindung in der Entwicklung einer Vorrichtung die zum Mischen des Knochenzementteigs aus den Ausgangskomponenten vorgesehen und geeignet ist sowie eines Verfahrens zur Herstellung eines Knochenzementteigs, insbesondere eines pastenförmigen Polymethylmethacrylat-Knochenzementteigs, wobei der Knochenzementteig aus einem Zementpulver und einer Monomerflüssigkeit hergestellt wird, mit denen die Nachteile der bisherigen Vorrichtungen und Verfahren überwunden werden. Die Erfindung hat die Aufgabe eine solche Vorrichtung derart zu verbessern, dass der fertiggestellte Knochenzementteig auch während des Austragens homogen bleibt. Zudem sollen in dem erzeugten Knochenzementteig möglichst keine Monomerblasen entstehen. Bei der Vorrichtung und dem Verfahren soll nach erfolgtem Monomertransfer beim Beginn und während des Austragens des gebildeten Knochenzementteigs eine Nachverdichtung der Glassplitter der geborstenen Glasampulle wirksam vermieden werden, wodurch ein nachträgliches Einspritzen von Monomerflüssigkeit in den Knochenzementteig verhindert werden soll. Eine Nachkompression der Glassplitter muss reproduzierbar ausgeschlossen werden. Ferner soll der Knochenzementteig in einer möglichst genau reproduzierbaren Konsistenz und Qualität zur Verfügung gestellt werden.

Mit der erfindungsgemäßen Vorrichtung und dem erfindungsgemäßen Verfahren soll also erreicht werden, dass auch bei einem sehr einfachen und kostengünstigen Aufbau der Vorrichtung und bei gleichzeitig sehr einfacher und unkomplizierter Anwendbarkeit der Vorrichtung von Anfang bis Ende des Auspressvorgangs ein homogener Knochenzementteig erzeugt und appliziert werden kann.

Die Vorrichtung soll durch eine einfache herkömmliche Auspressvorrichtung anzutreiben sein und dabei möglichst einfach in der Bedienung sein. Der Aufbau soll kostengünstig sein, damit die Vorrichtung aus hygienischen Gründen nur einmalig verwendet werden kann. Es sollen möglichst viele oder alle in der Vorrichtung ablaufenden Prozesse, wie die Vermischung der Ausgangskomponenten, das Öffnen des Monomerflüssigkeitsbehälters und möglichst auch das Austragen des Knochenzementteigs und gegebenenfalls auch das Öffnen der Kartusche mit möglichst wenigen Arbeitsschritten und so weit als möglich automatisiert ablaufen und vorzugsweise mit einem einzigen linearen Antrieb anzutreiben sein.

Die Handhabung der Vorrichtung soll dabei maximal vereinfacht sein, um Anwendungsfehler infolge von fehlerhaft durchgeführten Montageschritten grundsätzlich zu vermeiden. Der medizinische Anwender soll die Vorrichtung nach der Entnahme aus einer Verpackung mit einer Auspressvorrichtung verbinden und diese anschließend betätigen können. Weitere Montage- und Arbeitsschritte sollen durch den Aufbau der Vorrichtung vermieden werden. Die Vorrichtung soll vorzugsweise auch eine sichere Lagerung von Zementpulver und Monomerflüssigkeit in voneinander getrennten Kompartimenten ermöglichen, so dass während der Lagerung der Vorrichtung eine unbeabsichtigte Vermischung der Zementkomponenten ausgeschlossen ist. Die Vorrichtung soll eine Sterilisation mit dem Gas Ethylenoxid ermöglichen. Das in der Vorrichtung gelagerte Zementpulver muss dazu gegebenenfalls für Ethylenoxid zugänglich sein. Die Vorrichtung soll mit Hilfe einer im OP manuell angetriebenen Auspressvorrichtung aktivierbar sein, so dass nach der form- oder kraftschlüssigen Verbindung der Vorrichtung mit der Auspressvorrichtung durch Betätigung der Auspressvorrichtung die axial vortreibbare Stange der Auspressvorrichtung auf die Vorrichtung einwirkt, den Monomerflüssigkeitsbehälter öffnet und anschließend bei weiterer Bewegung der Stange die Monomerflüssigkeit in das Zementpulver transferiert. Die Vermischung der Monomerflüssigkeit mit dem Zementpulver soll ohne einen von außen manuell zu bewegenden Mischer erfolgen. Es soll möglichst nur mit der Vorwärtsbewegung der Stange der Auspressvorrichtung die Vermischung der Zementkomponenten unter Bildung des Knochenzementteigs und vorzugsweise auch das Auspressen des gemischten Knochenzementteigs vorgenommen werden.

Die Aufgaben der Erfindung werden gelöst durch eine Vorrichtung zum Herstellen eines Knochenzementteigs aus einer Monomerflüssigkeit und einem Zementpulver als Ausgangskomponenten des Knochenzementteigs und zum Austragen des gemischten Knochenzementteigs, die Vorrichtung aufweisend:
1) eine Kartusche mit einem zylindrischen Innenraum, wobei im Innenraum der Kartusche ein in Richtung einer Vorderseite der Kartusche beweglicher Austragskolben in einer Rückseite der Kartusche angeordnet ist, eine Aufnahme, die sich entlang einer Längsrichtung erstreckt, wobei eine Vorderseite der Aufnahme mit der Rückseite der Kartusche verbunden ist,
2) einen Förderkolben, der in der Aufnahme angeordnet ist, wobei der Förderkolben in Längsrichtung der Aufnahme in Richtung zur Vorderseite der Aufnahme hin beweglich in der Aufnahme gelagert ist,
3) eine aufbrechbare Ampulle enthaltend die Monomerflüssigkeit, wobei die Ampulle zwischen dem Förderkolben und dem Austragskolben in der Aufnahme angeordnet ist und wobei die Ampulle einen Ampullenkörper aufweist, wobei der Ampullenkörper zumindest bereichsweise an der Innenwand der Aufnahme anliegt, und
4) einen Abstandhalter, der in der Aufnahme zwischen dem Austragskolben und dem Ampullenkörper oder zwischen dem Förderkolben und dem Ampullenkörper angeordnet ist, wobei der Abstandhalter sich in Längsrichtung der Aufnahme erstreckt und wobei der Abstandhalter von der Innenwand der Aufnahme mit einem Abstand mindestens so groß wie die Wandstärke des Ampullenkörpers beabstandet ist.

Theoretisch kann erfindungsgemäß auch ein Abstandhalter zwischen dem Austragskolben und dem Ampullenkörper und ein zweiter Abstandhalter zwischen dem Förderkolben und dem Ampullenkörper angeordnet sein. Dann wird die Ampulle zwischen den beiden Abstandhaltern zerdrückt. Es also erfindungsgemäß nicht genau ein Abstandhalter vorgesehen, da auch noch ein weiterer (zweiter) Abstandhalter vorgesehen sein kann. Der Abstandhalter oder die Abstandhalter können dabei auch aus mehreren Teilen aufgebaut sein. Es ist jedoch erfindungsgemäß bevorzugt, wenn der Abstandhalter einteilig ist beziehungsweise die Abstandhalter einteilig sind.

Ebenso kann erfindungsgemäß auch mehr als eine Ampulle enthaltend die Monomerflüssigkeit zwischen dem Förderkolben und dem Austragskolben in der Aufnahme angeordnet sein. Die Ampullen werden dann nacheinander oder parallel aufgebrochen.

Die erfindungsgemäße Vorrichtung ist zum Lagern der Monomerflüssigkeit geeignet und vorgesehen. Bevorzugt ist die erfindungsgemäße Vorrichtung auch zum Lagern des Zementpulvers vorgesehen.

Die Aufnahme, die Kartusche, der Förderkolben, der Austragskolben und der Abstandhalter sind vorzugsweise aus einem thermoplastischen Kunststoff gefertigt, insbesondere mit einem Spritzgussverfahren. Hierdurch kann die Vorrichtung kostengünstig als hygienisches Wegwerfprodukt gefertigt werden.

Der Innenraum der Kartusche hat eine zylindrische Geometrie. Die zylindrische Form ist die einfachste, mit der sich der Innenraum der Kartusche realisieren lässt. Unter einer zylindrischen Form ist geometrisch die Form eines allgemeinen Zylinders mit einer beliebigen Grundfläche zu verstehen, also nicht nur ein Zylinder mit einer kreisförmigen Grundfläche. Die Innenwand des Innenraums der Kartusche kann also durch den Zylindermantel eines Zylinders mit beliebiger Grundfläche realisiert sein, insbesondere mit unterschiedlicher Grundfläche realisiert sein, das heißt auch mit nicht kreisförmigen oder nicht runden Grundflächen. Erfindungsgemäß wird jedoch eine zylindrische Geometrie mit rotationssymmetrischer und insbesondere kreisrunder Grundfläche für den Innenraum bevorzugt, da diese am einfachsten zu fertigen ist. Das gleiche gilt für den vorzugsweise ebenfalls zylindrisch geformten Innenraum der Aufnahme.

Bei erfindungsgemäßen Vorrichtungen kann vorgesehen sein, dass der Abstandhalter an der Rückseite des Austragskolbens oder an der Vorderseite des Förderkolbens befestigt ist oder dass der Abstandhalter um einen Ampullenkopf der Ampulle herum angeordnet ist, wobei der Ampullenkopf einen kleineren Außendurchmesser als der Ampullenkörper hat.

Wenn der Abstandhalter an der Rückseite des Austragskolbens oder an der Vorderseite des Förderkolbens befestigt ist, kann die Vorrichtung besonders leicht aufgebaut werden. Zudem ist hierdurch die Position des Abstandhalters festgelegt und er kann nicht verkippen, so dass eine stabile und sichere Bewegung des Abstandhalters gegen die Ampulle gewährleistet ist. Theoretisch kann auch ein Abstandhalter an der Rückseite des Austragskolbens und ein zweiter Abstandhalter an der Vorderseite des Förderkolbens befestigt sein. Je nachdem, an welchem Kolben (Förderkolben oder Austragskolben) der Abstandhalter befestigt ist (oder wenn die Abstandhalter an den beiden Kolben befestigt sind), können keine Splitter der Ampulle zwischen den jeweiligen Kolben und den Abstandhalter gelangen und so den Abstandhalter verkippen und/oder den Abstand zwischen den beiden Kolben verändern, der durch den Abstandhalter begrenzt werden soll.

Des Weiteren kann vorgesehen sein, dass an der der Ampulle zugewandten Vorderseite des Förderkolbens und/oder an der der Ampulle zugewandten Rückseite des Austragskolbens zumindest ein Spaltelement mit einer Schneidkante angeordnet ist, vorzugsweise zumindest drei oder zumindest vier Spaltelemente mit jeweils einer Schneidkante angeordnet sind, wobei das zumindest eine Spaltelement an einem zur Innenwand der Aufnahme liegenden Bereich der Vorderseite des Förderkolbens und/oder der Rückseite des Austragskolbens angeordnet ist, so dass das zumindest eine Spaltelement beim Vortreiben des Förderkolbens durch die Wandung des Ampullenkörpers schneidet, wobei sich bevorzugt die Schneidkante radial von einer zentralen Längsachse des Förderkolbens und/oder des Austragskolbens weg erstreckt.

Hierdurch wird erreicht, dass die Ampulle beziehungsweise der Ampullenkörper an definierten Stellen bricht beziehungsweise geschnitten wird. Dadurch lässt sich der Vorgang des Aufbrechens besser steuern und der Ablauf standardisieren. Damit können Splitter der Ampulle in vorhersagbarer Größe erzeugt werden, die gut in den durch den Abstandhalter erzeugten Raum passen. Dass das zumindest eine Spaltelement an einem zur Innenwand der Aufnahme liegenden Bereich der Vorderseite des Förderkolbens und/oder der Rückseite des Austragskolbens angeordnet ist, bedeutet nicht, dass sich das zumindest eine Spaltelement nicht auch bis zur Mitte der Vorderseite des Förderkolbens und/oder der Rückseite des Austragskolbens erstrecken kann. Es muss dann aber mindestens auch in einem äußeren, zur Innenwand der Aufnahme liegenden Bereich angeordnet sein, so dass es in der Längsrichtung mit der Wandung des Ampullenkörpers fluchtet. Durch die Anwendung einer definierten Kraft an einer vorherbestimmten und räumlich begrenzten Stelle kann der Druck an dieser Stelle bei gleicher Kraft erhöht werden und so ein definiertes Brechen der Ampulle erreicht werden. Dadurch wird der Ablauf des Aufbrechens der Ampulle reproduzierbarer.

Bevorzugt liegt das zumindest eine Spaltelement außen an der Innenwand der Aufnahme an. Es kann vorgesehen sein, dass ein freies Ende des Abstandhalters von dem zumindest einen Spaltelement beabstandet ist, vorzugsweise mit einem Abstand in Längsrichtung von mindestens 10 mm beabstandet ist.

Bei erfindungsgemäßen Vorrichtungen mit Spaltelemente kann vorgesehen sein, dass die Ampulle einen Ampullenkopf mit einem kleineren Durchmesser als der Ampullenkörper aufweist und dass das zumindest eine Spaltelement und der Abstandhalter an der Rückseite des Austragskolbens angeordnet sind, wobei der Ampullenkopf in Richtung des Austragskolbens weist, oder dass das zumindest eine Spaltelement und der Abstandhalter an der Vorderseite des Förderkolbens angeordnet sind, wobei der Ampullenkopf in Richtung des Förderkolbens weist.

Dadurch kann die Ampulle zuverlässig geöffnet werden. Zudem verläuft die durch das zumindest eine Spaltelemente erzeugte Brechfront beim Öffnen der Ampulle hinter den freien Enden des Abstandhalters, so dass die Splitter der Ampulle nicht zwischen die freien Enden des Abstandhalters und dem sich darauf zu bewegenden Förderkolben oder Austragskolben geraten und dadurch den durch den Abstandhalter bewirkten Abstand zwischen den zusammengeschobenen Austragskolben und Förderkolben ungewollt vergrößern.

Es kann erfindungsgemäß vorgesehen sein, dass der Ampullenkörper der Teil der Ampulle mit dem größten Querschnitt senkrecht zur Längsrichtung der Aufnahme ist. Hiermit wird sichergestellt, dass die Ampulle ein großes Fassungsvermögen hat und die Vorrichtung einigermaßen kompakt aufgebaut werden kann, das heißt, in der Längsrichtung nicht zu lang ist.

Ferner kann vorgesehen sein, dass der Abstandhalter in Längsrichtung wenigstens dreimal so lang ist, wie das zumindest eine Spaltelement in Längsrichtung von der Vorderseite des Förderkolbens oder der Rückseite des Austragskolbens absteht, wobei vorzugsweise der Abstandhalter in Längsrichtung wenigstens fünfmal so lang ist, wie das zumindest eine Spaltelement in Längsrichtung von der Vorderseite des Förderkolbens oder der Rückseite des Austragskolbens absteht.

Hierdurch wird erreicht, dass die durch das zumindest eine Spaltelemente erzeugte Brechfront beim Öffnen der Ampulle ausreichend weit hinter den freien Enden des Abstandhalters verläuft, so dass die Splitter der Ampulle nicht oder nicht so leicht zwischen die freien Enden des Abstandhalters und dem sich darauf zu bewegenden Förderkolben oder Austragskolben geraten und dadurch den durch den Abstandhalter bewirkten Abstand zwischen den zusammengeschobenen Austragskolben und Förderkolben ungewollt vergrößern.

Gemäß einer bevorzugten Weiterbildung der erfindungsgemäßen Vorrichtung kann vorgesehen sein, dass der Abstandhalter beim Vortreiben des Förderkolbens eine weitere Verringerung des Abstands des Förderkolbens zum Austragskolben nach dem Aufbrechen der Ampulle und nach einer Komprimierung der Ampulle blockiert, so dass Splitter der Ampulle zwischen dem Austragskolben und dem Förderkolben Platz finden ohne in kleinere Splitter zerbrochen zu werden, wenn der Förderkolben und der Austragskolben mit dem Abstandhalter dazwischen in Richtung der Vorderseite der Kartusche bewegt werden.

Die Splitter der Ampulle können bei der Komprimierung der Ampulle bereits mehrfach zerbrochen worden sein (auch zeitlich nacheinander), bevor sie nicht mehr in (noch) kleinere Splitter zerbrochen werden.

Hierdurch wird erreicht, dass, nachdem der Abstandhalter den Abstand zwischen dem Förderkolben und dem Austragskolben festgelegt hat, keine weitere Komprimierungen beim gemeinsamen Vortreiben des Förderkolbens und des Austragskolbens durch später brechende Splitter mehr auftreten können, die den Zwischenraum zwischen dem Förderkolben und dem Austragskolben weiter verkleinern und dadurch weitere Monomerflüssigkeit in den Innenraum der Kartusche drücken, die dann entweder als Monomerblasen in den Knochenzementteig gelangt oder die die Konsistenz des Knochenzementteigs während des Auspressvorgangs verändert.

Vorzugsweise kann auch vorgesehen sein, dass der Abstandhalter eine Länge in der Längsrichtung hat, so dass das Volumen zwischen dem Förderkolben und dem Austragskolben bei einem Abstand, der der Länge des Abstandhalters entspricht, größer ist als das Volumen des Materials der Ampulle, vorzugsweise mindestens doppelt so groß ist wie das Volumen des Materials der Ampulle oder mindestens so groß ist wie das Volumen der Splitter der zerbrochenen Ampulle einschließlich aller Zwischenräume.

Hierdurch kann sichergestellt werden, dass die Glassplitter nicht mit sehr großer Kraft komprimiert werden müssen, beziehungsweise die beim Auspressen des Knochenzementteigs bei gemeinsamer Bewegung des Förderkolbens und des Austragskolbens auftretende Kraft nicht ein weiteres Zersplittern der Splitter der Ampulle bewirkt, die zu einer weiteren Verringerung des Volumens zwischen dem Förderkolben und dem Austragskolben führt und dadurch ungewollt zusätzliche Monomerflüssigkeit in den Knochenzementteig in dem Innenraum der Kartusche presst. Hiermit kann ein homogener Knochenzementteig sichergestellt werden.

Mit einer bevorzugten Weiterbildung der vorliegenden Erfindung wird vorgeschlagen, dass der Abstandhalter durch eine Mehrzahl von Stäben realisiert ist, die sich in Längsrichtung erstrecken, wobei die Stäbe miteinander verbunden sind oder an der Vorderseite des Förderkolbens befestigt sind oder an der Rückseite des Austragskolbens befestigt sind, wobei die Stäbe vorzugsweise im Querschnitt rund, dreieckig oder eckig, insbesondere viereckig, sind und/oder im Längsschnitt T-förmig geformt sind.

Die Stäbe nehmen selbst nicht viel Volumen ein und können den Abstand zwischen dem Austragskolben und dem Förderkolben an wenigstens zwei Punkten stabilisieren. Zudem können die Stäbe vorzugsweise an deren freien Enden spitz zulaufen, so dass diese sich gut durch die Wandung der Ampulle vor dem Ampullenkörper bohren können. Zudem können Splitter der Ampulle leicht von den Stäben zur Seite gedrängt werden. Bevorzugt ist der Abstandhalter durch wenigstens drei Stäbe realisiert, besonders bevorzugt durch drei, vier, fünf oder sechs Stäbe.

Erfindungsgemäß kann auch vorgesehen sein, dass der Abstandhalter durch einen Hohlzylinder realisiert ist, insbesondere durch einen in Längsrichtung mehrfach geschlitzten Hohlzylinder.

Des Weiteren kann vorgesehen sein, dass die Ampulle einen Ampullenkopf aufweist, der mit dem Ampullenkörper verbunden ist, wobei der Ampullenkopf einen kleineren Außendurchmesser als der Ampullenkörper hat und wobei der Abstandhalter neben dem Ampullenkopf angeordnet ist oder der Abstandhalter den Ampullenkopf umgibt.

Hiermit kann die Bewegung des Abstandhalters auf einfache Weise festgelegt werden und der Aufbrechvorgang standardisiert werden. Zudem wird so die Ampulle in der Vorrichtung stabil gehalten und gelagert.

Ferner kann vorgesehen sein, dass die Ampulle aus Glas oder aus einem gegenüber der Monomerflüssigkeit chemisch stabilen Kunststoff besteht, wobei Glas als Material für die Ampulle bevorzugt ist.

Diese Materialien sind gut zur Lagerung der Monomerflüssigkeit geeignet.

Es kann auch vorgesehen sein, dass der Ampullenkörper zylindrisch geformt ist und die Aufnahme in ihrem Inneren zylindrisch geformt ist, wobei vorzugsweise der Außendurchmesser des Ampullenkörpers zum Innendurchmesser der im Inneren zylindrisch geformten Aufnahme passt, so dass der Ampullenkörper in der Aufnahme gehalten ist, insbesondere flächenbündig gehalten ist.

Hierdurch wird die Ampulle stabil in der Vorrichtung gelagert, so dass sie nicht versehentlich durch Stöße vorzeitig in der Vorrichtung aufbrechen kann.

Bevorzugt kann vorgesehen sein, dass der Förderkolben von einer Rückseite der Aufnahme aus in der Längsrichtung zur Vorderseite vortreibbar gelagert ist.

Hierdurch kann die Vorrichtung in eine Auspressvorrichtung, wie eine Kartuschenpistole, eingespannt und mit dieser betrieben werden.

Es kann vorgesehen sein, dass der Innenraum der Kartusche an der Vorderseite bis auf eine Austragsöffnung zum Austreiben des Knochenzementteigs verschlossen ist, wobei der Austragskolben im Innenraum der Kartusche in Richtung der Austragsöffnung drückbar ist.

Dadurch kann der Knochenzementteig durch diese Austragsöffnung ausgepresst werden.

Dabei kann vorgesehen sein, dass eine Austragsöffnung der Kartusche an deren Vorderseite mit einem Verschluss, insbesondere mit einem Stopfen, verschlossen ist, wobei der Knochenzementteig durch die Austragsöffnung aus der Kartusche auspressbar ist, wenn die Austragsöffnung geöffnet ist, und wobei vorzugsweise der Verschluss für Gase durchlässig und für das Zementpulver undurchlässig ist. Der Verschluss ist vorzugsweise ein für Gase durchlässiger und für das Zementpulver undurchlässiger Filter, insbesondere Porenfilter.

Hierdurch kann das Zementpulver gut in dem Innenraum der Kartusche gelagert werden. Der Verschluss kann geöffnet werden. Der Innenraum der Kartusche und das Zementpulver können durch Evakuieren und Spülen des Innenraums der Kartusche mit einem sterilisierenden Gas, wie Ethylenoxid, durch den Verschluss hindurch sterilisiert werden, wenn dieser für Gase durchlässig und für das Zementpulver undurchlässig ist.

Dabei kann vorgesehen sein, dass der Verschluss an der dem Innenraum der Kartusche zugewandten Rückseite eine Vertiefung aufweist, in der der vorderste Teil des Zementpulvers enthalten ist. Dieser Teil kann dann später mit dem Verschluss entfernt werden, so dass ein weniger gut durchmischter Teil des Knochenzementteigs mit dem Verschluss entfernt wird. Der Verschluss bildet vorzugsweise mit dem Austragskolben ein durch axialen Druck auf den Austragskolben in Richtung der Austragsöffnung wirkenden Druck zu öffnendes Verschlusssystem der Kartusche.

Bei einer Weiterbildung der vorliegenden Erfindung kann vorgesehen sein, dass die Rückseite der Kartusche mit der Vorderseite der Aufnahme derart verbunden ist, dass der Innenraum der Kartusche mit dem Innenraum der Aufnahme fluchtet.

Hiermit wird sichergestellt, dass der Förderkolben gemeinsam mit dem Austragskolben in den Innenraum der Kartusche vorgetrieben werden kann, um den Knochenzementteig aus dem Innenraum der Kartusche auszupressen.

Bevorzugt kann auch vorgesehen sein, dass das Zementpulver im Innenraum der Kartusche zwischen der Vorderseite der Kartusche und dem Austragskolben angeordnet ist, wobei vorzugsweise in dem Zementpulver ein die Monomerflüssigkeit leitendes Additiv verteilt ist.

Dadurch ist die Vorrichtung gleich einsatzbereit und muss nicht vorher mit dem Zementpulver befüllt werden.

Es kann dabei auch vorgesehen sein, dass das Zementpulver an der Vorderseite des Austragskolbens anliegt, insbesondere vollflächig anliegt, wobei vorzugsweise das Zementpulver in den Innenraum der Kartusche eingepresst ist.

Hierdurch wird verhindert, dass in der Kartusche größere Gaseinschlüsse verbleiben, die beim Mischen der Monomerflüssigkeit mit dem Zementpulver zu Gaseinschlüssen im Knochenzementteig führen könnten. Dies kann bei einem dicht geschütteten Zementpulver nicht passieren, da die Monomerflüssigkeit die Partikel des Zementpulvers gut benetzt und die Oberflächenspannung der Monomerflüssigkeit dann keine oder zumindest keine relevanten Gaseinschlüsse zwischen den Partikeln des Zementpulvers erlaubt.

Es kann auch vorgesehen sein, dass das Volumen der Zwischenräume zwischen den Zementpartikeln des Zementpulvers im Innenraum der Kartusche im Bereich von 22 Volumenprozent bis 40 Volumenprozent bezogen auf das Gesamtvolumen des Zementpulvers ist. Das Gesamtvolumen des Zementpulvers entspricht bevorzugt dem Volumen des Innenraums der Kartusche, der durch den Austragskolben und durch einen Verschluss in einer Austragsöffnung an der Vorderseite der Kartusche begrenzt ist.

Des Weiteren kann vorgesehen sein, dass in dem Zementpulver ein die Monomerflüssigkeit leitendes Additiv verteilt ist, wobei vorzugsweise das Zementpulver mit dem Additiv beschichtet oder mit dem Additiv gemischt ist.

Als Additiv kann beispielsein biokompatibler Zellstoff verwendet werden, der eine ausreichende Saugfähigkeit für die Monomerflüssigkeit aufweist. Das Additiv kann partikulär in dem Zementpulver verteilt sein.

Hiermit kann erreicht werden, dass die Monomerflüssigkeit sich schnell in dem Zementpulver verteilt und so eine vollständige Mischung entsteht, bevor das anquellende Zementpulver eine weitere Ausbreitung der Monomerflüssigkeit verhindern würde. Dadurch ist es möglich, die Monomerflüssigkeit auch über längere Strecken durch das Zementpulver zu leiten und so einen homogenen Knochenzementteig zu erzeugen.

Ferner kann vorgesehen sein, dass in dem Zementpulver ein hydrophiles Additiv verteilt ist, mit dem die Monomerflüssigkeit in dem gesamten Zementpulver verteilbar ist, bevorzugt ohne dass zuvor eine Polymerisation des Knochenzements die weitere Verteilung der Monomerflüssigkeit in dem Zementpulver verhindert.

Hiermit wird erreicht, dass die Monomerflüssigkeit in dem Zementpulver schnell verteilt wird, bevor eine Polymerisation des in dem Zementpulver enthaltenen Zementpulvers mit der Monomerflüssigkeit stattfindet und dadurch eine weitere Verteilung der Monomerflüssigkeit unterbunden wird. Nur hierdurch ist der erfindungsgemäße Aufbau in einer gemeinsam mit der Aufnahme ausgeformten Kartusche überhaupt möglich, dass nämlich die Monomerflüssigkeit von einer Seite in das Zementpulver gepresst wird und sich dennoch in dem gesamten Zementpulver verteilen kann, bevor die Polymerisierung eine weitere Verteilung der Monomerflüssigkeit in dem Zementpulver unterbindet.

Das Additiv ist vorzugsweise Partikulär oder Faserförmig. Bevorzugt weist das Additiv eine chemische Substanz mit zumindest einer OH-Gruppe auf. Das Additiv hat bevorzugt ein Aufsaugvermögen von mindestens 0,6 g Methylmethacrylat pro Gramm Additiv.

Es kann erfindungsgemäß vorgesehen sein, dass das Zementpulver mindestens ein partikuläres Polymethylmethacrylat oder Polymethylmethacrylat-Copolymer der Siebfraktion kleiner 100 µm, einen Initiator, und mindestens ein in Methylmethacrylat unlösliches, partikuläres oder faserförmiges Additiv aufweist, wobei das Additiv ein Aufsaugvermögen größer gleich 0,6 g Methylmethacrylat pro Gramm Additiv bei Raumtemperatur besitzt.

Ein solches Zementpulver ist besonders gut zur Verteilung der Monomerflüssigkeit in dem Zementpulver geeignet, so dass ein Aufbau der Vorrichtung ermöglicht wird, mit dem ein einseitiges Einpressen der Monomerflüssigkeit auch auf einer Schmalseite des Innenraums der Kartusche möglich ist. Dabei wurde überraschend gefunden, dass es möglich ist, durch einfaches In-Kontakt-Bringen eines solchen Zementpulvers mit einer Monomerflüssigkeit einen klebfreien, plastisch verformbaren Knochenzementteig herzustellen, der selbstständig durch radikalische Polymerisation aushärtet, ohne dass es notwendig ist, den Zementteig manuell oder mit Hilfe von technischen Hilfsmitteln zu vermischen. Es wurde beobachtet, dass durch Zusatz eines in Methylmethacrylat unlöslichen, partikulären oderfaserförmigen Additivs, das ein Aufsaugvermögen von größer 0,6 g Methylmethacrylat pro Gramm Additiv bei Raumtemperatur besitzt, zu einem Zementpulver eines niedrig-viskosen Knochenzements, ein modifiziertes Zementpulver als Zementpulver erhalten wird, in das Monomerflüssigkeit über eine Strecke von mindestens 5 cm eingepresst werden kann. Das Additiv verbessert überraschend auch die Benetzung des Zementpulvers mit Monomerflüssigkeit. Das Additiv hat dabei einen "Docht-Effekt" und leitet schon in sehr geringen Mengen ab 0,1 Gew% die Monomerflüssigkeit in das Innere des Zementpulvers. Weiterhin verzögert das Additiv das Verkleben der Polymerpartikel im Zementpulver, wodurch die Ausbildung einer blockierenden Gelschicht verzögert wird und das Eindringen der Monomerflüssigkeit in das Zementpulver begünstigt wird. Die Monomerflüssigkeit kann dabei in das Zementpulver eingepresst oder auch eingesaugt werden.

Dabei kann bevorzugt vorgesehen sein, dass das Additiv kovalent gebundene Hydroxylgruppen an seiner Oberfläche besitzt. Das Additiv kann erfindungsgemäß bevorzugt ausgewählt sein aus der Gruppe, die aus mikrokristalliner Cellulose, Oxycellulose, Stärke, Titandioxid und Siliziumdioxid besteht, wobei pyrogenes Siliziumdioxid besonders bevorzugt wird. Das Additiv kann eine Partikelgröße der Siebfraktion kleiner 100 µm, bevorzugt der Siebfraktion kleiner 50 µm und ganz besonders bevorzugt von der Siebfraktion kleiner 10 µm aufweisen. Ferner kann bevorzugt vorgesehen sein, dass das Additiv im Zementpulver in einer Menge von 0,1 bis 2,5 Gew.% bezogen auf das Gesamtgewicht des Zementpulvers enthalten ist. Des Weiteren kann vorgesehen sein, dass das Polymerpulver Dibenzoylperoxid als Initiator enthält.

Es kann vorgesehen sein, dass die Monomerflüssigkeit mindestens ein Methylmethacrylat und einen Aktivator enthält. Des Weiteren kann vorgesehen sein, dass die Monomerflüssigkeit mindestens einen Aktivator aus der Gruppe der aromatischen Amine enthält. Ferner kann vorgesehen sein, dass die Monomerflüssigkeit mindestens einen radikalischen Stabilisator aus der Gruppe der Chinone oder der sterisch gehinderten Phenole enthält.

Vorteilhaft ist es, wenn das Additiv kovalent gebundene Hydroxylgruppen an seiner Oberfläche besitzt. Besonders vorteilhaft sind dabei besonders Si-OH-Gruppen und alkoholische OH-Gruppen. Durch die oberflächlich angeordneten OH-Gruppen hat das Additiv eine hohe Oberflächenenergie, wodurch eine gute Benetzbarkeit des Additivs mit Methylmethacrylat erreicht wird. Die pyrogenen Kieselsäuren Aerosil® 380 und Aerosil® 300 sind besonders geeignet. Daneben ist es auch möglich, durch Sol-Gel-Prozesse erzeugtes Siliziumdioxid als Additiv zu verwenden.

Mit der Erfindung wird auch vorgeschlagen, dass an der Vorderseite des Austragskolbens ein Hohlzylinder angeordnet ist, der eine weitere Bewegung des Austragskolbens in Richtung der Vorderseite der Kartusche blockiert, so dass der Austragskolben bereichsweise von der Vorderseite des Innenraums der Kartusche beabstandet ist und ein Totvolumen in dem Innenraum der Kartusche verbleibt, wenn der Austragskolben gegen die Vorderseite des Innenraums der Kartusche gedrückt ist.

Hiermit kann verhindert werden, dass ein weniger gut durchmischter Teil des Knochenzementteigs, der sich in der Nähe des Austragskolbens befindet, am Ende des Auspressvorgangs ausgepresst werden kann. Zudem kann entlang des Hohlzylinders die Monomerflüssigkeit in einen tieferen Bereich in ein Zementpulver im Innenraum der Kartusche geleitet werden.

Dabei kann vorgesehen sein, dass das Totvolumen zumindest 1 cm³ groß ist, bevorzugt zumindest 3 cm³ groß ist.

Mit einer Weiterbildung der vorliegenden Erfindung kann vorgesehen sein, dass in dem Austragskolben zumindest eine für die Monomerflüssigkeit und Gase durchlässige aber für das Zementpulver undurchlässige Verbindung vorgesehen ist, die die Vorderseite des Austragskolbens mit der Rückseite des Austragskolbens verbindet oder dass ein Innenraum der Aufnahme und der Innenraum der Kartusche über eine für die Monomerflüssigkeit und für Gase durchlässige aber für das Zementpulver undurchlässige Verbindung miteinander verbunden sind. Hierdurch können der Innenraum der Kartusche und der Innenraum der Aufnahme mit einem sterilisierenden Gas wie Ethylenoxid sterilisiert werden. Zudem kann verhindert werden, dass das Zementpulver in die Verbindung vordringt, dort nach dem Öffnen der Ampulle vorzeitig mit der Monomerflüssigkeit reagiert und dadurch die Verbindung durch anquellenden Knochenzementteig verschließt und so eine weitere Durchleitung der Monomerflüssigkeit in das Zementpulver behindert oder verhindert.

Es kann erfindungsgemäß auch vorgesehen sein, dass in der Wandung der Aufnahme zumindest eine Belüftungsöffnung angeordnet ist, die den Innenraum der Aufnahme mit der Umgebung verbindet. Dadurch kann die Vorrichtung auch im Inneren mit einem sterilisierenden Gas wie Ethylenoxid gespült oder sogar durchspült werden.

Dabei kann bevorzugt vorgesehen sein, dass die zumindest eine Belüftungsöffnung derart dicht im Bereich des Förderkolbens angeordnet ist, dass sie durch eine Bewegung des Förderkolbens in Richtung der Vorderseite der Aufnahme verschlossen wird, bevor die in der Aufnahme angeordnete Ampulle durch die Bewegung des Förderkolbens geöffnet ist. Hierdurch kann verhindert werden, dass die Monomerflüssigkeit aus der Aufnahme durch die zumindest eine Belüftungsöffnung nach außen fließen kann.

Es kann zur Anwendung einer Auspressvorrichtung wie einer Kartuschenpistole auch vorgesehen sein, dass an der Rückseite der Vorrichtung, ein Befestigungsmittel zur Befestigung einer Auspressvorrichtung angeordnet ist, mit der der Förderkolben und Austragskolben in Richtung der Vorderseite der Kartusche drückbar ist.

Es kann auch vorgesehen sein, dass an der Vorderseite der Kartusche ein Austragsrohr angeordnet, wobei der Knochenzementteig durch das Austragsrohr auspressbar ist. Hiermit kann der Knochenzementteig bequemer appliziert werden.

Bevorzugt kann ferner vorgesehen sein, dass das Volumen der Monomerflüssigkeit in der Ampulle mindestens so groß ist wie das Volumen der mit Luft gefüllten Zwischenräume zwischen den Zementpulverpartikeln in der Kartusche, bevorzugt mindestens so groß ist wie das Volumen der Flüssigkeitsleitungen zwischen dem Innenraum der Kartusche und dem Innenraum der Aufnahme plus das Volumen zwischen dem Förderkolben und dem Austragskolben bei einem Abstand, der der Länge des Abstandhalters entspricht, abzüglich des Volumens des Materials der Ampulle, plus das Volumen der mit Luft gefüllten Zwischenräume zwischen den Zementpulverpartikeln in der Kartusche. Hiermit wird sichergestellt, dass genug Monomerflüssigkeit zur Bildung des Knochenzementteigs verfügbar ist.

Die der vorliegenden Erfindung zugrundeliegenden Aufgaben werden auch gelöst durch ein Verfahren zur Herstellung eines Knochenzementteigs, insbesondere eines pastenförmigen Polymethylmethacrylat-Knochenzementteigs, wobei der Knochenzementteig aus einem Zementpulver und einer Monomerflüssigkeit hergestellt wird, gekennzeichnet durch die folgenden Schritte:
A) zwischen einem in einer Längsrichtung beweglichen Förderkolben und einem Austragskolben sind eine Ampulle enthaltend die Monomerflüssigkeit und ein sich in einer Längsrichtung erstreckender Abstandhalter in einer Aufnahme angeordnet, wobei der Förderkolben in Längsrichtung zum Austragskolben hin gedrückt wird,
B) durch die Bewegung des Förderkolbens gegen den Austragskolben wird ein Ampullenkopf der Ampulle aufgebrochen oder abgebrochen, wobei ein freies Ende des Abstandhalters im Inneren der geöffneten Ampulle gegen einen Ampullenkörper der geöffneten Ampulle bewegt wird, so dass während der Bewegung zumindest ein Teil der Wandung des Ampullenkörpers zwischen dem Abstandhalter und einer Innenwand der Aufnahme angeordnet ist,
C) die geöffnete Ampulle wird durch die Bewegung des Förderkolbens gegen den Austragskolben komprimiert und weiter zerbrochen und die Monomerflüssigkeit wird dabei aus der Aufnahme heraus und in das Zementpulver hinein gepresst, wo sie sich mit dem Zementpulver unter Bildung des Knochenzementteigs vermischt,
D) der Abstandhalter wird zwischen dem Förderkolben und dem Austragskolben eingeklemmt und verhindert dadurch eine weitere Reduzierung des Abstands des Austragskolbens zum Förderkolben und damit ein weiteres Auspressen von Monomerflüssigkeit aus der Aufnahme in den Knochenzementteig.

Dabei kann vorgesehen sein, dass das Verfahren mit einer erfindungsgemäßen Vorrichtung durchgeführt wird.

Das Verfahren hat dadurch die Vorteile der erfindungsgemäßen Vorrichtung.

Des Weiteren kann vorgesehen sein, dass in Schritt C) die Monomerflüssigkeit durch zumindest eine für das Zementpulver undurchlässige aber für Gase und die Monomerflüssigkeit durchlässige Verbindung im Austragskolben in eine Kartusche enthaltend das Zementpulver hineingepresst wird.

Hierdurch kann verhindert werden, dass das Zementpulver zuvor in die Verbindung vordringt, dort mit der Monomerflüssigkeit reagiert und dabei die Verbindung verschließt, so dass keine weitere Monomerflüssigkeit in das Zementpulver gedrückt werden kann.

Ferner kann vorgesehen sein, dass die Bewegung des Förderkolbens in Schritt B) und C) durch eine axiale Bewegung einer Stange einer Auspressvorrichtung angetrieben wird, die vor Schritt A) an der Aufnahme befestigt wird.

Dadurch kann das Verfahren beziehungsweise die Bewegung des Förderkolbens mit einer herkömmlichen Auspressvorrichtung, wie einer Auspresspistole, angetrieben werden.

Auch kann vorgesehen sein, dass an der Rückseite des Austragskolbens oder an der Vorderseite des Förderkolbens zumindest ein Spaltelement mit einer Schneidkante angeordnet ist, wobei in Schritt C) die Wandung des Ampullenkörpers mit der Schneidkante geschnitten oder gebrochen wird, wobei das freie Ende des Abstandhalters von dem zumindest einen Spaltelement beabstandet ist, vorzugsweise mit einem Abstand in Längsrichtung von mindestens 10 mm beabstandet ist.

Hierdurch wird erreicht, dass möglichst keine Splitter der Ampulle zwischen das freie Ende des Abstandhalters und den Austragskolben oder den Förderkolben gelangen. Dadurch kann sichergestellt werden, dass der Abstand zwischen dem Förderkolben und dem Austragskolben in Schritt D) sehr genau eingestellt werden kann und dadurch die Menge der in das Zementpulver eingetragenen Monomerflüssigkeit sehr genau vorbestimmt werden kann. Dadurch kann die gewünschte Konsistenz des Knochenzementteigs sehr präzise eingestellt werden.

Schließlich wird auch vorgeschlagen, dass das Zementpulver in einem Innenraum einer Kartusche angeordnet ist, wobei der Austragskolben in Längsrichtung beweglich in dem Innenraum der Kartusche angeordnet ist, wobei in Schritt C) die Monomerflüssigkeit in den Innenraum der Kartusche gepresst wird und wobei nach Schritt D) in einem Schritt E) der Austragskolben von dem Förderkolben in Längsrichtung in den Innenraum der Kartusche gedrückt wird und dabei der Knochenzementteig aus dem Innenraum der Kartusche herausgedrückt wird.

Hierdurch kann durch die lineare Bewegung des Förderkolbens auch der Austragskolben angetrieben werden und der Knochenzementteig mit der gleichen Bewegung und damit mit dem gleichen linearen Antrieb ausgetragen werden, der auch zum Einpressen der Monomerflüssigkeit in das Zementpulver verwendet wird.

Es kann dabei vorgesehen sein, dass in Schritt E) durch den auf den Knochenzementteig wirkenden Druck ein Verschluss, insbesondere ein Porenfilter, in einer Austragsöffnung an der Vorderseite der Kartusche bewegt oder hervorgedrückt wird, wobei bevorzugt hierauf der Verschluss aus der Austragsöffnung entfernt wird und besonders bevorzugt danach ein Applikationsrohr an der Vorderseite der Kartusche befestigt wird.

Der Erfindung liegt die überraschende Erkenntnis zugrunde, dass es durch den von der Innenwand der Aufnahme beabstandeten Abstandhalter gelingt, den minimalen Abstand zwischen dem Förderkolben und dem Austragskolben derart präzise einzustellen, dass die Menge der aus der Aufnahme in das Zementpulver transferierten Monomerflüssigkeit sehr genau eingestellt werden kann und gut reproduzierbar ist. Dadurch, dass sich ein freies Ende des Abstandhalters beim Zusammenpressen im Inneren des Ampullenkörpers der geöffneten Ampulle bewegt, kann die Brechfront oder die Schneidfront, die durch den Ampullenkörper läuft, von dem freien Ende des Abstandhalters beabstandet werden, so dass vermieden werden kann (oder zumindest die Gefahr reduziert werden kann), dass Bruchstücke der Ampulle (also Scherben der Ampulle) zwischen die freien Enden des Abstandhalters und die gegenüberliegende Vorderseite des Förderkolbens oder die gegenüberliegende Rückseite des Austragskolbens gelangen. Hiermit bestimmt die Länge des Abstandhalters in Längsrichtung den Abstand zwischen dem Austragskolben und dem Förderkolben, wenn der Förderkolben derart weit zum Austragskolben gedrückt ist, dass der Abstandhalter zwischen dem Förderkolben und dem Austragskolben eingeklemmt ist. Allenfalls der Boden der Ampulle befindet sich dann noch zwischen dem freien Ende des Abstandhalters und der Vorderseite des Förderkolbens oder der Rückseite des Austragskolbens, wobei vorzugsweise auch dieser zersplittert wird. Dadurch ist das Volumen zwischen dem Förderkolben und dem Austragskolben im zusammengedrückten Zustand sehr genau bekannt. Aufgrund des bekannten Volumens des Abstandhalters und des bekannten Volumens der Wandungen der Ampulle, beziehungsweise der Scherben der Ampulle, kann die in dem Volumen zurückbleibende Menge der Monomerflüssigkeit aus der Ampulle sehr genau vorhergesagt werden und damit auch die Menge der in das Zementpulver gepressten Monomerflüssigkeit. Auch das Volumen einer gegebenenfalls vorhandenen Lagerung als Transportsicherung für die Ampulle in der Aufnahme ist bekannt. Dadurch ist es möglich, den Knochenzementteig reprozierbar mit der gewünschten Konsistenz mit der erfindungsgemäßen Vorrichtung und dem erfindungsgemäßen Verfahren herzustellen, indem eine definierte Menge der Monomerflüssigkeit in das Zementpulver gepresst wird.

Besonders vorteilhaft ist es, wenn zumindest ein Spaltelement mit einer Schneidkante vorgesehen sind, die den Ampullenkörper an einer definierten Stelle mechanisch aufbrechen oder aufschneiden, die von dem freien Ende des Abstandhalters möglichst weit beabstandet ist. Die Brechfront oder Schneidfront, an der die Bruchstücke beziehungsweise Scherben entstehen, ist dann möglichst weit von dem freien Ende des Abstandhalters entfernt.

Die Erfindung beruht auch darauf, dass bei Bewegung des Förderkolbens in Richtung des Austragskolbens zuerst der Ampullenkopf in den zylinderförmigen Ampullenkörper (den Glasmantel) der Ampulle gedrückt wird oder zuerst der mindestens eine Abstandhalter in den Ampullenkörper gedrückt wird. Gleichzeitig dringt der mindestens eine Abstandhalter in das Innere des zylinderförmigen Ampullenkörpers der Ampulle ein, ohne dass der Ampullenkörper durch den mindestens einen Abstandhalter zerstört wird. Bei weiterer Bewegung des Förderkolbens in Richtung des Austragskolbens werden der Abstandhalter und gegebenenfalls der Ampullenkopf in den Hohlraum des Ampullenkörpers geschoben und andererseits trifft die Vorderseite des Förderkolbens oder bevorzugt das mindestens eine Spaltelement auf der Vorderseite des Förderkolbens auf die Wandung des Ampullenkörpers (den Glasmantel der Glasampulle) und wirkt auf diesen ein. Alternativ kann auch die Rückseite des Austragskolbens oder bevorzugt das wenigstens eine Spaltelement auf der Rückseite des Austragskolbens auf die Wandung des Ampullenkörpers einwirken. Die Wandung beginnt bei weiterer Bewegung des Förderkolbens in Richtung des Austragskolbens zu zerbrechen. Das bedeutet, die Bildung der Bruchstücke (der Glassplitter) verläuft hinter der Vorwärtsbewegung des Abstandhalters. Der Abstandhalter läuft dann auf den Austragskolben oder den Förderkolben auf und verhindert die weitere Bewegung des Förderkolbens in Richtung des Austragskolbens. Dieser Prozess ist nur präzise möglich, weil die Bildung der Glassplitter nachfolgend dem vorauseilenden freien Ende des Abstandhalters erfolgt. Es können sich dadurch keine Splitter zwischen dem Abstandhalter und den Austragskolben oder den Förderkolben schieben.

Kurz vor dem Auflaufen des Abstandhalters gleitet der Abstandhalter, wenn er durch mehrere Stäbe realisiert ist, bevorzugt erfindungsgemäß am Boden der Ampulle nach außen in Richtung Kartuschenwand ab. Das bedeutet, dass bei weiterer Bewegung des Förderkolbens durch den Abstandhalter der Austragskolben synchron in Richtung Kartuschenkopf mit bewegt wird, ohne dass sich der Abstand zwischen dem Austragskolben und dem Förderkolben ändern kann. Dass bedeutet, die axiale Länge des Abstandhalters (in Längsrichtung) definiert eindeutig den Abstand zwischen dem Austragskolben und dem Förderkolben. Voraussetzung dafür ist, dass der Abstandhalter mechanisch stabil, insbesondere torsions- und knickstabil, ausgebildet ist. Eine elastische Verformung des Abstandhalters kann aber vorgesehen sein.

Die bevorzugte erfindungsgemäße Vorrichtung mit Zementpulver in dem Innenraum der Kartusche hat die wesentlichen Vorteile, dass die beiden Ausgangskomponenten des Knochenzementteigs im geschlossenen Zementiersystem gelagert sind und dass die Vermischung der Ausgangskomponenten in der geschlossenen Vorrichtung erfolgt. Das bedeutet, dass die Vorrichtung nicht vom Anwender befüllt werden muss. Es handelt sich dann um ein Full-Prepacked-Zementiersystem. Der medizinische Anwender hat keinerlei Kontakt mit den einzelnen Ausgangskomponenten der Knochenzemente. Die Geruchsbelästigung ist dadurch nur noch minimal.

Ein besonderer Vorteil der Vorrichtung besteht auch darin, dass durch das einfache Vorwärtsbewegen einer Stange einer manuell angetriebenen Auspressvorrichtung die Monomerflüssigkeit in das Zementpulver gepresst wird. Dabei wird die zwischen den Zementpulverpartikeln vorhandene Luft durch die Monomerflüssigkeit verdrängt. Es entsteht ein homogener Knochenzementteig, ohne dass ein manuelles Mischen mit Mischstäben mit Mischflügeln notwendig ist. Das bedeutet, dass das fehlerbehaftete manuelle Mischen nicht mehr notwendig ist. Die Bedienung der Vorrichtung ist maximal vereinfacht. Es handelt sich um ein Ready-to-use-System.

Die Vorteile erfindungsgemäßer Vorrichtungen und Verfahren beruhen auch darauf, dass die an sich bekannte lineare Vorwärtsbewegung von Stangen von manuell betriebenen Auspressvorrichtungen so genutzt werden, dass durch kontinuierliche Einwirkung der Kraft der linearen Vorwärtsbewegung der Stange zuerst ein Monomerflüssigkeitsbehälter geöffnet wird, der Monomerflüssigkeitsbehälter anschließend zusammengepresst wird, wodurch die Monomerflüssigkeit aus dem Monomerflüssigkeitsbehälter austritt und in ein vorzugsweise verdichtetes Zementpulver eingepresst wird, wobei die zwischen den Zementpulverpartikeln vorhandene Luft durch die eingepresste Monomerflüssigkeit verdrängt wird und nach Benetzung der Zementpulverpartikel durch die Monomerflüssigkeit ein Knochenzementteig entsteht. Voraussetzung dafür ist die Verwendung eines Zementpulvers, das so eingestellt ist, dass es sehr gut von der Monomerflüssigkeit benetzt wird und diese durch Kapillarwirkung einsaugen kann.

Die Vorrichtung kann als hygienisches Wegwerfprodukt verwendet werden, da sie sehr weitgehend aus Kunststoff gefertigt werden kann und weil alle Teile einschließlich der Innenräume und des Zementpulvers mit Hilfe von Ethylenoxid sterilisierbar sind.

Eine beispielhafte erfindungsgemäße Vorrichtung zum Lagern, Vermischen und Austragen von Polymethylmethacrylat-Knochenzement kann beispielsweise aufweisen:
eine hohlzylinderförmige Kartusche, einen axial beweglichen Förderkolben, einen für Gase und Flüssigkeiten durchlässigen für Pulverpartikel jedoch undurchlässigen axial beweglichen Austragskolben, einen ersten Hohlraum mit einer darin angeordneten Glasampulle enthaltend eine Monomerflüssigkeit, einen zweiten Hohlraum mit darin angeordnetem Zementpulver und einen für Gase durchlässigen, für Pulverpartikel undurchlässigen, axial beweglichen Verschlussstopfen, mindestens ein keilförmiges Spaltelement, das radial so angeordnet ist, dass der Glasmantel der Glasampulle auf dem gleichen Radius bezogen auf die Längsachse der Kartusche liegt, mindestens ein Abstandhalter, der sich parallel zur Längsachse der Kartusche zwischen dem Austragskolben und dem Förderkolben erstreckt, wobei der Abstandhalter einen Ampullenkopf der Glasampulle umgibt und auf einen Radius, der kleiner als der Radius der Innenseite des Glasmantels der Glasampulle ist, angeordnet ist, wobei der Abstandhalter den Austragskolben zum Förderkolben derart axial (in Längsrichtung) beabstandet, dass das Volumen des ersten Hohlraums, durch den Förderkolben, den für Gase und Flüssigkeiten durchlässigen und für Pulverpartikel undurchlässigen Austragskolben und der inneren Kartuschenwand gebildet wird, größer gleich dem Volumen der Glassplitter der geborstenen Glasampulle ist.

Der Abstandhalter und das Spaltelement können entweder zusammen am Austragskolben oder beide alternativ am Förderkolben angeordnet sein.

Weiterhin ist es möglich, den mindestens einen Abstandhalter und bevorzugt auch das mindestens eine Spaltelement auf einem Ring oder ringförmig anzuordnen und diesen vor dem Förderkolben oder hinter dem Austragskolben anzuordnen.

Es kann vorgesehen sein, dass der Abstandhalter mehrere Stäbe umfasst, die an der Rückseite des Austragskolbens oder an der Vorderseite des Förderkolbens befestigt sind und sich in Längsrichtung in der Aufnahme erstrecken. Dabei kann vorgesehen sein, dass die Stäbe zusätzlich über wenigstens einen Ring miteinander verbunden sind, wobei der wenigstens eine Ring von der Rückseite des Austragskolbens oder der Vorderseite des Förderkolbens beabstandet ist. Hierdurch werden die Stäbe stabilisiert und stabilisieren sich gegeneinander. Dadurch kann für die Stäbe ein normaler (unverstärkter) Kunststoff verwendet werden, da sich die Stäbe über den wenigstens einen Ring gegenseitig stabilisieren. Die Stäbe und der wenigstens eine Ring sind vorzugsweise einteilig aus Kunststoff gefertigt.

Erfindungsgemäß kann vorgesehen sein, dass das der Abstandhalter und das mindestens eine Spaltelement am Förderkolben, auf der dem Austragskolben zugewandten Vorderseite des Förderkolbens, angeordnet sind, wenn die Ampulle mit dem Ampullenkopf in Richtung des Förderkolbens ausgerichtet ist.

Weiterhin kann erfindungsgemäß vorgesehen sein, dass der Abstandhalter und das mindestens eine Spaltelement an dem für Gase und Flüssigkeiten durchlässigen und für Pulverpartikel undurchlässigen Austragskolben, auf der dem Förderkolben zugewandten Rückseite des Austragskolbens, angeordnet sind, wenn die Ampulle mit dem Ampullenkopf in Richtung des Austragskolbens ausgerichtet ist.

Der Abstandhalter wird bevorzugt durch drei oder vier Stäbe gebildet, wobei die Stäbe rund, dreieckig, viereckig oder T-förmig sind. Die Stäbe können als Hohlkörper oder als volle Körper ausgebildet sein. Die Stäbe können aus Kunststoffen, Polyamid, Polyamid, Polyketon, Polyethersulfon und Polyimiden und bevorzugt aus Glasfasere-verstärkten Kunstoffen bestehen. Daneben ist es auch möglich, dass die Stäbe aus Stahl, Titan und Titanlegierungen bestehen.

Erfindungsgemäß kann bevorzugt auch vorgesehen sein, dass der Abstandhalter durch einen Holzylinder oder durch Teile eines Hohlzylinders gebildet wird.

Der Abstandhalter ist bevorzugt mindestens fünf mal so hoch wie das Spaltelement in axialer Richtung. Dadurch wird gewährleistet, dass die Bildung der Glassplitter immer der Bewegung des freien Endes des Abstandhalters nachläuft.

Bevorzugt stehen alle Spaltelemente des wenigstens einen Spaltelements maximal 2 mm von der Vorderseite des Förderkolbens oder der Rückseite des Austragskolbens ab, vorzugsweise maximal 1 mm.

Im Folgenden werden weitere Ausführungsbeispiele der Erfindung anhand von sechzehn schematisch dargestellten Figuren erläutert, ohne jedoch dabei die Erfindung zu beschränken. Dabei zeigt:
Figur 1: eine schematische Querschnittansicht einer beispielhaften ersten erfindungsgemäßen Vorrichtung zum Herstellen eines Knochenzementteigs;
Figur 2: eine schematische Seitenansicht der ersten erfindungsgemäßen Vorrichtung nach Figur 1;
Figur 3: eine schematische perspektivische Querschnittansicht der ersten erfindungsgemäßen Vorrichtung nach den Figuren 1 und 2;
Figur 4: fünf schematische Querschnittansichten der ersten erfindungsgemäßen Vorrichtung nach den Figuren 1 bis 3 mit einer angeschlossenen Auspressvorrichtung übereinander zur Verdeutlichung des Ablaufs eines erfindungsgemäßen Verfahrens;
Figur 5: eine schematische Querschnittansicht der ersten erfindungsgemäßen Vorrichtung nach den Figuren 1 bis 4 nach dem Auspressen des erzeugten Knochenzementteigs;
Figur 6: drei schematische perspektivische Ansichten auf erfindungsgemäße Vorrichtungen nach den Figuren 1 bis 5 mit Applikationsrohr, ohne Aufsatz und mit Kappe am Austragsrohr;
Figur 7: eine schematische Querschnittansicht als Ausschnittvergrößerung durch den vorderen Teil der ersten erfindungsgemäßen Vorrichtung nach Figur 1 im Ausgangszustand;
Figur 8: drei schematische Querschnittansichten als Ausschnittvergrößerungen der ersten erfindungsgemäßen Vorrichtung nach den Figuren 1 bis 7 im Ausgangszustand, nach Aufbrechen der Ampulle und während des Brechens des Ampullenkörpers, wie in der ersten, der dritten und der vierten Abbildung von oben in Figur 3 dargestellt ist;
Figur 9: eine perspektivische Querschnittansicht (oben) und zwei weitere perspektivische Ansichten (Mitte und unten) des Austragskolbens der ersten erfindungsgemäßen Vorrichtung nach den Figuren 1 bis 8;
Figur 10: eine schematische Querschnittansicht einer beispielhaften zweiten erfindungsgemäßen Vorrichtung zum Herstellen eines Knochenzementteigs;
Figur 11: eine schematische perspektivische Querschnittansicht der zweiten erfindungsgemäßen Vorrichtung nach Figur 10;
Figur 12: fünf schematische Querschnittansichten der zweiten erfindungsgemäßen Vorrichtung nach den Figuren 10 und 11 mit einer angeschlossenen Auspressvorrichtung übereinander zur Verdeutlichung des Ablaufs eines erfindungsgemäßen Verfahrens;
Figur 13: eine schematische Querschnittansicht als Ausschnittvergrößerung der zweiten erfindungsgemäßen Vorrichtung nach der ersten Abbildung von oben von Figur 12, die mit der Auspressvorrichtung verbunden ist;
Figur 14: eine schematische Querschnittansicht als Ausschnittvergrößerung der zweiten erfindungsgemäßen Vorrichtung nach der dritten Abbildung von oben von Figur 12, beim Aufbrechen der Ampulle;
Figur 15: eine schematische Querschnittansicht als Ausschnittvergrößerung des vorderen Teils der zweiten erfindungsgemäßen Vorrichtung nach den Figuren 10 bis 14 nach dem Auspressen des Knochenzementteigs, mit aufgesetzter Austragsspitze; und
Figur 16: eine perspektivische Ansicht (oben), eine Seitenansicht (Mitte) und eine Querschnittansicht (unten) des Förderkolbens der zweiten erfindungsgemäßen Vorrichtung nach den Figuren 10 bis 15;

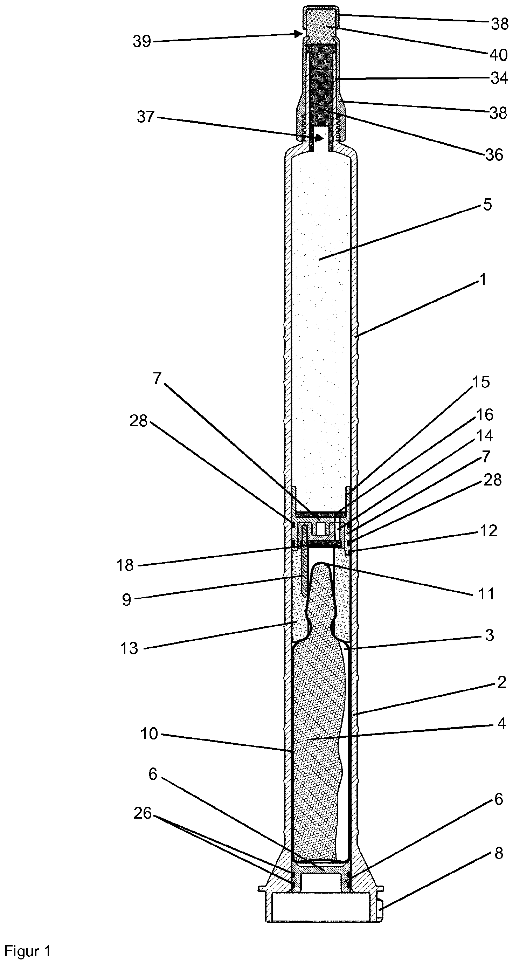

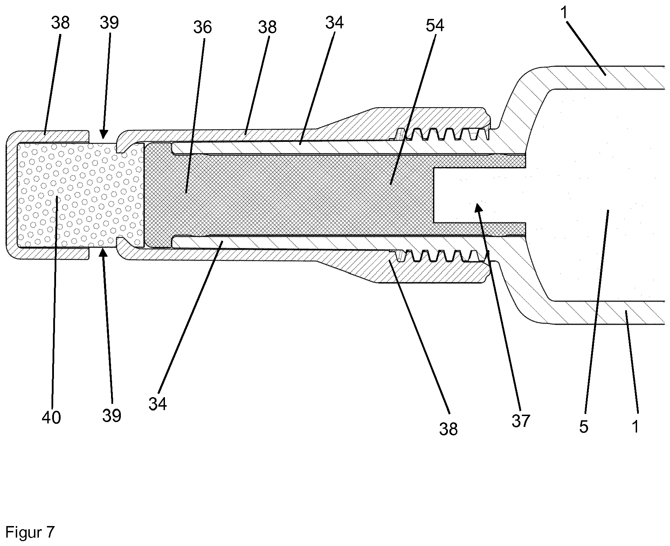

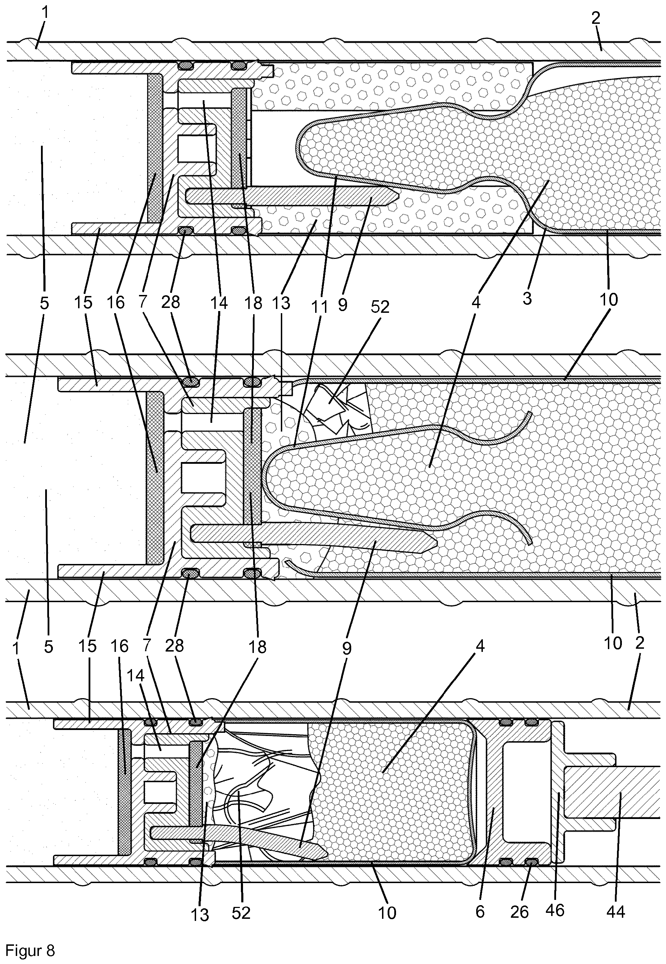

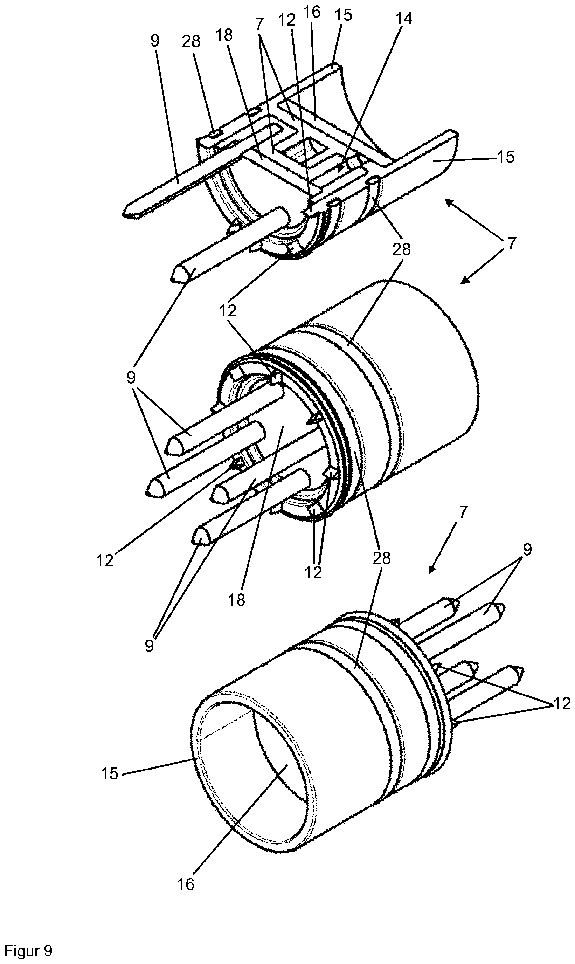

In den Figuren 1 bis 9 sind Abbildungen einer ersten erfindungsgemäßen Vorrichtung zum Lagern und Mischen eines Knochenzementteigs gezeigt. Die Figuren 1 bis 6 zeigen verschiedene schematische Gesamtansichten der ersten beispielhaften erfindungsgemäßen Vorrichtung. Die Figuren 7 und 8 zeigen Ausschnittvergrößerungen von schematischen Querschnittansichten als Detailansichten durch verschiedene Bereiche der ersten erfindungsgemäßen Vorrichtung und Figur 9 zeigt drei schematische Detailansichten eines Austragskolbens der ersten erfindungsgemäßen Vorrichtung.

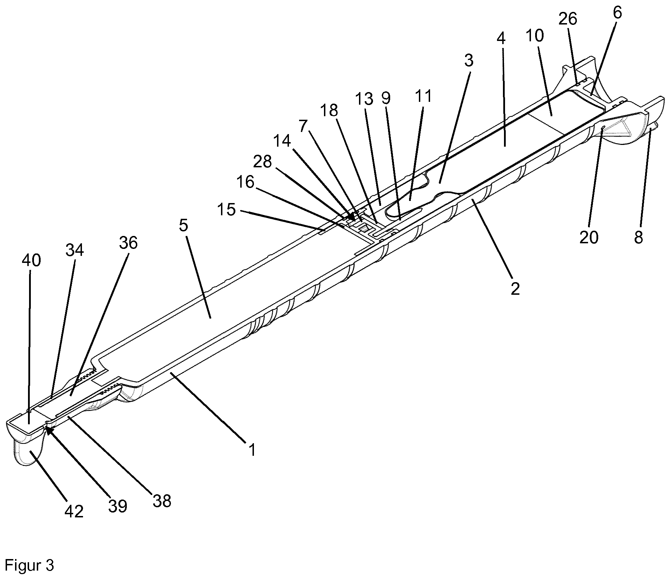

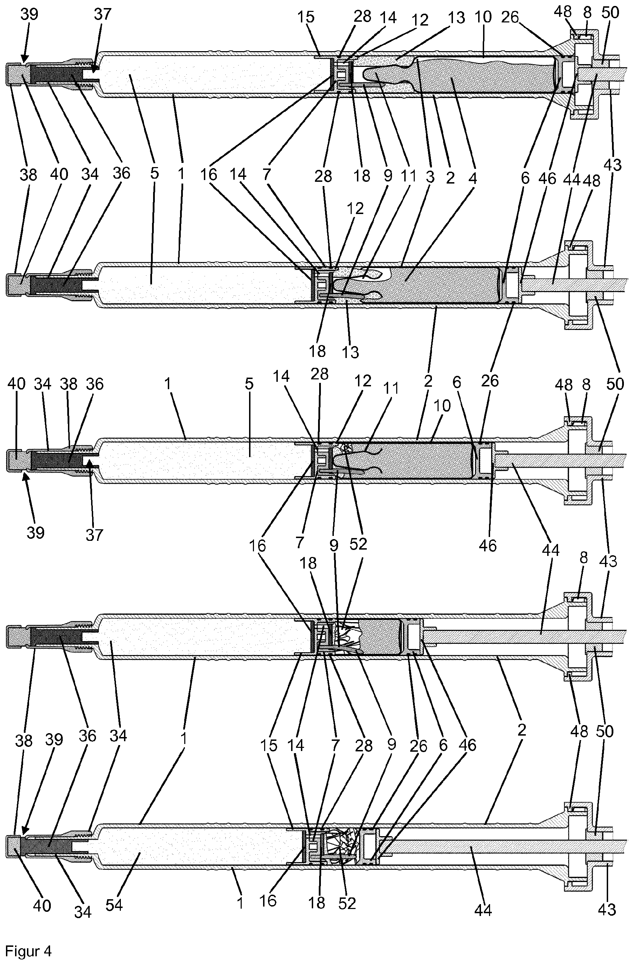

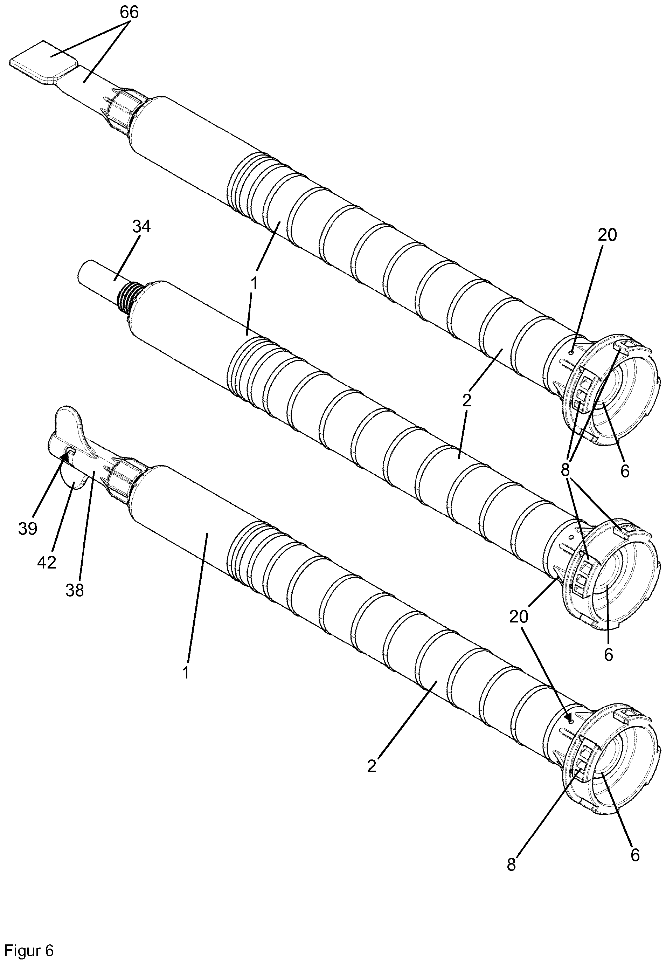

Die erste erfindungsgemäße Vorrichtung besteht im Wesentlichen aus einem röhrenförmigen Behälter aus Kunststoff, der als vorderen Teil (in den Figuren 1, 2 und 5 oben, in Figur 3 links unten, in den Figuren 4, 7 und 8 links und in Figur 6 links oben) eine Kartusche 1 mit einem zylindrischen Innenraum bildet und der als hinteren Teil eine Aufnahme 2 mit einem zylindrischen Innenraum für eine Glasampulle 3 (oder eine Kunststoffampulle 3) als Behälter für eine Monomerflüssigkeit 4 bildet. Die Rückseite der Vorrichtung ist in den Figuren 1, 2 und 5 unten, in Figur 3 rechts oben, in Figur 4 rechts und in Figur 6 rechts unten gezeigt. Die Röhrenform des Behälters ist besonders gut in den Querschnittansichten der Figuren 1, 3 und 5 zu erkennen. Sowohl der Innenraum der Kartusche 1 als auch der Innenraum der Aufnahme 2 sind zylindrisch mit kreisförmiger Grundfläche. Dabei sind die Durchmesser des Innenraums der Kartusche 1 und der Durchmesser des Innenraums der Aufnahme 2 gleich groß und fluchten miteinander. Der Behälter mit der Aufnahme 2 und der Kartusche 1 wird vorzugsweise mit Hilfe einer Spritzgusstechnik aus Kunststoff hergestellt. Die Aufnahme 2 weist also einen zylindrischen Innenraum auf, in den die Glasampulle 3 eingesteckt ist. In der Glasampulle 3 befindet sich die Monomerflüssigkeit 4. In den Innenraum der Kartusche 1 ist ein Zementpulver 5 eingefüllt oder vorzugsweise eingepresst. Die Monomerflüssigkeit 4 und das Zementpulver 5 bilden die Ausgangskomponenten für einen PMMA-Knochenzement, der mit der Vorrichtung hergestellt werden kann. Aufgrund der Glasampulle 3 kann die Monomerflüssigkeit 4 sehr lange in der Aufnahme 2 und dadurch in der Vorrichtung gelagert werden. Das Zementpulver 5 kann ebenfalls über längere Zeiträume in der Vorrichtung gelagert werden. Die Vorrichtung ist damit zum Lagern der Monomerflüssigkeit 4 und des Zementpulvers 5 als Ausgangskomponenten eines Knochenzementteigs des PMMA-Knochenzements geeignet. Die Vorrichtung ist aber auch zum Mischen des Knochenzementteigs aus den Ausgangskomponenten und zum Austragen des gemischten Knochenzementteigs geeignet und vorgesehen.

In der Aufnahme 2 ist ein im zylindrischen Innenraum der Aufnahme 2 in Längsrichtung beweglicher Förderkolben 6 aus Kunststoff angeordnet. Der Förderkolben 6 ist im Bereich der Rückseite der Aufnahme 2 angeordnet. Die Glasampulle 3 kann mit dem Förderkolben 6 in der Aufnahme 2 zusammengedrückt und dabei zersplittert werden, indem der Förderkolben 6 in Richtung der Vorderseite, also in Richtung der Kartusche 1 gedrückt wird. Der Förderkolben 6 weist an der Vorderseite Abstreifer auf, mit denen Splitter der Glasampulle 3 von der Innenwand der Aufnahme 2 abgestreift werden. Die Abstreifer liegen hierzu seitlich an der Innenwand des Innenraums der Aufnahme an.

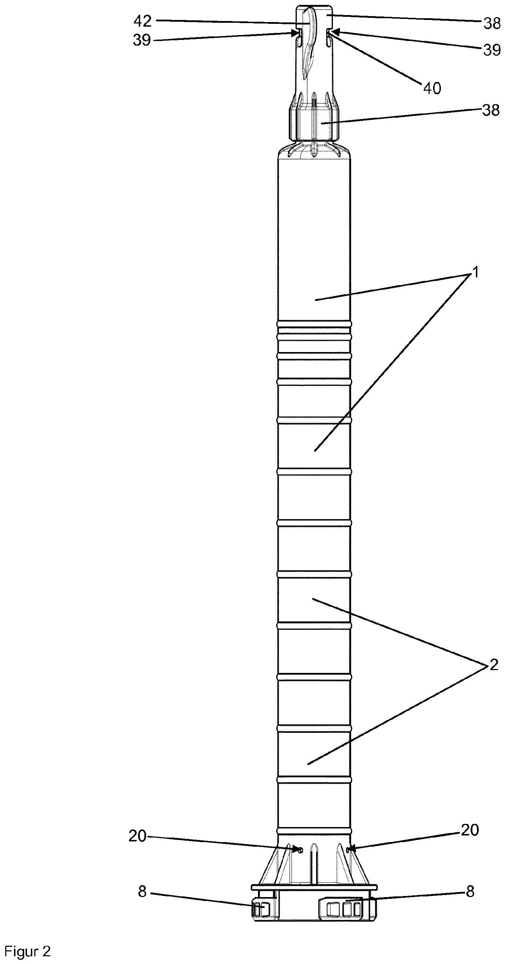

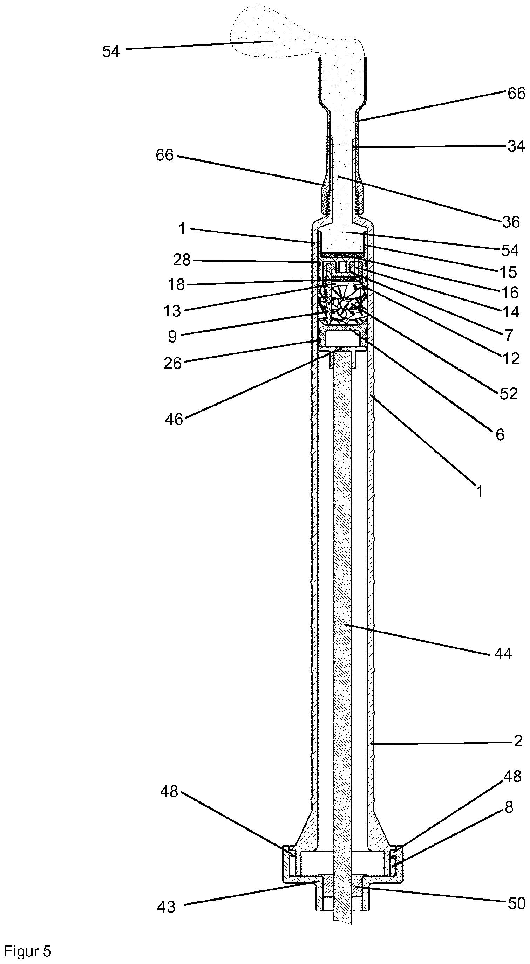

In dem Innenraum der Kartusche 1 ist in dessen Rückseite (in den Figuren 1 und 2 Richtung unten, in Figur 3 nach rechts oben, in den Figuren 4 und 8 Richtung rechts) ein Austragskolben 7 aus Kunststoff angeordnet, der in den Abbildungen nach Figur 9 im Detail dargestellt ist. An der Rückseite der Aufnahme 2 ist ein Befestigungsmittel 8 vorgesehen, mit dem die Aufnahme 2 an einer Auspressvorrichtung 43 (in den Figuren 1 bis 3 nicht zu sehen, siehe aber die Figuren 4 und 5) verbunden werden kann. Das Befestigungsmittel 8 ist vorzugsweise zur Bildung eines Bajonettverschlusses 8 geeignet und vorgesehen. Dadurch kann der Förderkolben 6, der von der Rückseite der Aufnahme 2 aus frei zugänglich ist, mit der Auspressvorrichtung 43 in Richtung der Vorderseite der Kartusche 1 vorgetrieben werden.

Der Austragskolben 7 weist an seiner Rückseite vier Stäbe 9 als Abstandhalter auf, die den Abstand zwischen dem Austragskolben 7 und dem Förderkolben 6 bestimmen, wenn der Förderkolben 6 vollständig in Richtung des Austragskolbens 7 gedrückt ist (siehe Figur 5). Die Stäbe 9 sind rotationssymmetrisch (beispielsweise zylindrisch), können aber auch einen eckigen Querschnitt aufweisen. Die Stäbe 9 erstrecken sich mindestens 10 mm von der Rückseite des Austragskolbens 7 in die Aufnahme 2 hinein. Die Stäbe 9 sind in Richtung des Förderkolbens 6 zulaufend geformt, wobei die Spitzen ein stumpfes Ende aufweisen (siehe Figur 9). Durch die spitz zulaufende Spitze der Stäbe 9 können zwischen den Spitzen der Stäbe 9 und dem Förderkolben 6 entstehende Splitter 52 beim Zerdrücken der Glasampulle 3 durch die Bewegung des Förderkolbens 6 leichter seitlich an den Stäben 9 vorbeigedrückt werden. Durch das stumpfe Ende der Stäbe 9 wird verhindert, dass sich die Stäbe 9 in den Förderkolben 6 eindrücken oder die Stäbe 9 an der Spitze verformt werden und dadurch sich die Länge der Stäbe 9 verändert und dadurch der Abstand zwischen dem Austragskolben 7 und dem Förderkolben 6 und damit der Raum dazwischen variiert und weniger gut vorherbestimmbar wird. Dadurch ist die in dem Zwischenraum zwischen dem Austragskolben 7 und dem Förderkolben 6 verbleibende Menge der Monomerflüssigkeit 4 nach dem Auspressen (siehe Figur 5) und damit die in das Zementpulver 5 eingepresste Menge der Monomerflüssigkeit 4 sehr genau bekannt und vorhersehbar. Dadurch ist die Konsistenz des erzeugten Knochenzementteigs 54 sehr genau einstellbar und reproduzierbar.

Die Glasampulle 3 weist einen Ampullenkörper 10 und einen Ampullenkopf 11 auf, die über einen dünnen Hals miteinander verbunden sind. Die Glasampulle 3 kann sehr einfach durch Abbrechen des Ampullenkopfs 11 vom Ampullenkörper 10 geöffnet werden. Die Stäbe 9 verlaufen seitlich vom Ampullenkopf 11 und umgeben diesen (siehe Figuren 1, 3 und 4), so dass bei einer Bewegung der Glasampulle 3 durch Vortreiben des Förderkolbens 6 die Stäbe 9 seitlich am Ampullenkopf 11 vorbeilaufen und in die Schultern der Glasampulle 3 in den Ampullenkörper 10 hineingetrieben werden (siehe Figuren 4 und 8). Die Stäbe 9 sind elastisch verformbar und aus einem Kunststoff gefertigt. Durch die elastische Verformbarkeit können Bruchstücke der Glasampulle 3 leichter an den Stäben 9 vorbeigeleitet werden. Die Stäbe 9 sind von der Innenwand der Aufnahme 2 zumindest so weit beabstandet, dass die Wandung des Ampullenkörpers 10 zwischen die Stäbe 9 und die Innenwand der Aufnahme 2 passt. Dadurch verlaufen die Stäbe 9 beim Vortreiben des Förderkolbens 6 im Inneren des Ampullenkörpers 10.

Es kann vorgesehen sein, dass die Stäbe 9 über einen gemeinsamen Ring (nicht gezeigt) miteinander verbunden sind. Der Ring ist parallel zur Rückseite des Austragskolbens 7 angeordnet und kann beispielsweise mit der halben Länge der Stäbe 9 von der Rückseite des Austragskolbens 7 beabstandet sein. Hierdurch werden die Stäbe 9 stabilisiert und können weniger leicht abknicken. Je länger die Stäbe 9 sind, desto vorteilhafter ist eine Stabilisierung mit einem Ring (oder auch mit mehreren Ringen). Der Innendurchmesser des Rings muss dabei groß genug sein, um den Ampullenkopf 11 aufnehmen zu können.

An der Rückseite des Austragskolbens 7 sind acht keilförmige Spaltelemente 12 angeordnet, die zum Spalten oder Brechen des Ampullenkörpers 10 der Glasampulle 3 bei Vortreiben des Förderkolbens 6 vorgesehen sind. Dazu verlaufen die Kanten der Spaltelemente 12 radial nach außen und sind außen am Austragskolben 7 angeordnet, so dass die Kanten der Spaltelemente 12 durch die komplette Wandung des Ampullenkörpers 10 laufen können und diesen dabei zersplittern. Die Spitzen der Stäbe 9 sind von den Spaltelementen 12 in Längsrichtung der Vorrichtung beabstandet, so dass die Brechfront, an der der Ampullenkörper 10 zersplittert wird, von den Spitzen der Stäbe 9 beabstandet ist. Hierdurch kann vermieden werden, dass viele Splitter 52 zwischen den Spitzen der Stäbe 9 und dem Förderkolben 6 entstehen, die zwischen den Spitzen der Stäbe 9 und dem Förderkolben 6 eingeklemmt werden könnten und dadurch das minimale Volumen zwischen dem Förderkolben 6 und dem Austragskolben 7 und damit die in das Zementpulver 5 eingepresste Menge der Monomerflüssigkeit 4 beeinflussen können.

Zur stabilen Lagerung der Glasampulle 3 ist eine Lagerung 13 in Form einer Hülse aus Schaumstoff als Stoßsicherung vorgesehen. Die hülsenförmige Lagerung 13 umgibt den Ampullenkopf 11 und ist zwischen den Stäben 9 und der Innenwand der Aufnahme 2 eingesteckt. Die Lagerung 13 ist für die Monomerflüssigkeit 4 durchlässig und aus einem Kunststoff gefertigt.

Die Kartusche 1 und die Aufnahme 2 sind einteilig als gemeinsames Kunststoffteil ausgeführt. Die Aufnahme 2 und die Kartusche 1 sind für die Monomerflüssigkeit 4 flüssigkeitsdurchlässig miteinander über eine Verbindung 14 im Austragskolben 7 verbunden. An der Vorderseite des Austragskolbens 7 ist ein Hohlzylinder 15 angeordnet. Die Verbindung 14 durch den Austragskolben 7 mündet durch einen für das Zementpulver 5 undurchlässigen aber für die Monomerflüssigkeit 4 durchlässigen Porenfilter 16 in den Innenraum der Kartusche 1.

In der Einmündung zur Verbindung 14 ist in dem Austragskolben 7 ein Filter 18 angeordnet, mit dem die Splitter 52 der Glasampulle 3 zurückgehalten werden können. Statt dem Filter 18 oder zusätzlich zum Filter 18 kann auch ein Sieb vorgesehen sein.

Mehrere Belüftungsöffnungen 20 sind in der Wandung der Aufnahme 2 vorgesehen, durch die der Innenraum der Aufnahme 2 mit Hilfe eines sterilisierenden Gases wie Ethylenoxid sterilisiert werden kann. Die Belüftungsöffnungen 20 sind unmittelbar benachbart zum Förderkolben 6 angeordnet, so dass der Förderkolben 6 sich unmittelbar vor die Belüftungsöffnungen 20 schiebt und somit die Belüftungsöffnungen 20 unmittelbar verschließt, wenn der Förderkolben 6 in Richtung der Kartusche 1 vorgetrieben wird. Dadurch wird verhindert, dass Monomerflüssigkeit 4 durch die Belüftungsöffnungen 20 austreten kann, wenn die Glasampulle 3 in der Aufnahme 2 geöffnet wurde.

Der zylindrische Förderkolben 6 hat einen zur Zylindergeometrie des Innenraums der Aufnahme 2 passenden Außenumfang und ist über zwei umlaufende Dichtungen 26 gegen die Innenwand der Aufnahme 2 flüssigkeitsdicht abgedichtet. Ebenso ist der Austragskolben 7 über zwei umlaufende Dichtungen 28 gegen die Innenwand der Kartusche 1 flüssigkeitsdicht abgedichtet. Diese Dichtungen 26, 28 dienen dazu, einen Austritt von Monomerflüssigkeit 4 oder von Knochenzementteig 54 zu verhindern, um eine Kontamination der Umgebung (des OP-Saals und des Anwenders) zu verhindern. Die Dichtungen 26, 28 können hierzu aus Gummi bestehen.

Der Innenraum der Kartusche 1 mündet an der Vorderseite in ein Austragsrohr 34, das eine vordere Austragsöffnung der Kartusche 1 begrenzt. Das Austragsrohr 34 weist an seiner Basis ein Außengewinde auf. Im Inneren der Austragsrohrs 34 ist ein Porenfilter 36 als Verschluss für die Kartusche 1 angeordnet. Der Porenfilter 36 ist für das Zementpulver 5 undurchlässig aber für Gase durchlässig. In der Rückseite des Porenfilters 36 ist eine Vertiefung 37 vorgesehen. Das Zementpulver 5 ist auch in der Vertiefung 37 enthalten. An dem Außengewinde des Austragsrohrs 34 ist eine Kappe 38 befestigt, wobei der vordere Teil der Kappe 38 mit einem Styropor oder Schaumstoff 40 gefüllt ist. An der Kappe 38 sind zwei Flügel 42 vorgesehen, so dass die Kappe 38 nach Art einer Flügelschraube bequem von dem Austragsrohr 34 abgeschraubt werden kann. Die Kappe 38 weist seitliche Öffnungen 39 auf. Durch diesen Aufbau kann das Innere der Kartusche 1 und das Zementpulver 5 mit Hilfe von Ethylenoxid sterilisiert werden, da die Öffnungen 39 in der Kappe 38, das Styropor oder der Schaumstoff 40, der Porenfilter 36 und die Zwischenräume zwischen den Pulverpartikeln des Zementpulvers 5 luftdurchlässig sind. Gleichzeitig kann Luft aus der Aufnahme 2 durch das Zementpulver 5, den Porenfilter 36, das Styropor oder der Schaumstoff 40 und die Öffnungen 39 in der Kappe 38 herausgepresst werden, wenn der Förderkolben 6 in Richtung der Aufnahme 1 gepresst wird. Die Kappe 38 bildet zusammen mit dem Styropor oder Schaumstoff 40 und mit dem Porenfilter 36 einen Verschluss für die Austragsöffnung der Kartusche 1 beziehungsweise für das Austragsrohr 34.

Das Zementpulver 5 ist in der Kartusche 1 eingeschlossen, da alle Öffnungen 39 und Verbindungen 14 mit Hilfe der Porenfilter 16, 36 für das Zementpulver 5 undurchlässig verschlossen sind. Der Inhalt der Kartusche 1 kann dabei durch Evakuieren und Spülen mit Ethylenoxid sterilisiert werden. Dadurch ist die Vorrichtung auch zum langfristigen Lagern des Zementpulvers 5 geeignet.

Figur 4 zeigt fünf schematische Querschnittansichten der ersten erfindungsgemäßen Vorrichtung nach den Figuren 1 bis 9 übereinander zur Verdeutlichung des Ablaufs eines erfindungsgemäßen Verfahrens. Als letzter Schritt des Verfahrens wird schließlich der Zustand in Figur 5 erreicht. Dazu zeigt Figur 7 eine Ausschnittvergrößerung der obersten Abbildung der Figur 4 und die drei Abbildungen von Figur 8 zeigen Ausschnittvergrößerungen der ersten Abbildung, der dritten Abbildung und der vierten Abbildung von oben der Figur 4.

Zu Beginn des Verfahrens liegt die Vorrichtung im Ausgangszustand vor, so wie sie auch in den Figuren 1 bis 3 gezeigt ist. In diesem Zustand wird die Vorrichtung in eine erfindungsgemäße Auspressvorrichtung 43 eingesetzt, die weitgehend einer herkömmlichen Kartuschenpistole entspricht. Diese Situation ist in der obersten Abbildung von Figur 4 gezeigt. Die Auspressvorrichtung 43 umfasst eine linear vortreibbare Stange 44. Von der Auspressvorrichtung 43 ist nur der vordere Teil dargestellt. Die Auspressvorrichtung 43 umfasst auch einen Griff und einen Kipphebel (in den Abbildungen nicht zu sehen) zum manuellen Antreiben der Stange 44 der Auspressvorrichtung 43, wie bei herkömmlichen manuell angetriebenen Auspressvorrichtungen 43 auch. Die Vorrichtung wird mit dem Befestigungsmittel 8 an der Auspressvorrichtung 43 befestigt (siehe oberste Abbildung in Figur 4). An der Spitze der Stange 44 ist ein flacher Teller 46 zum Antreiben des Förderkolbens 6 vorgesehen. Die Stange 44 drückt mit dem Teller 46 auf den Förderkolben 6, wenn die Stange 44 von der Auspressvorrichtung 43 in die Aufnahme 2 hineingedrückt wird. Die Auspressvorrichtung 43 ist hierzu über ein Gegenbefestigungsmittel 48 an die Rückseite der Aufnahme 2 angeschlossen, so dass der Teller 46 beim Vortreiben der Stange 44 auf den Förderkolben 6 drückt und diesen in Richtung der Kartusche 1 vortreibt. Hierzu ist die Stange 44 gegen ein Lager 50 und darüber gegen das Gegenbefestigungsmittel 48 und damit gegen die Aufnahme 2 linear beweglich gelagert.

Die Auspressvorrichtung 43 wird bedient und dabei die Stange 44 und mit der Stange 44 der Förderkolben 6 in Richtung der Kartusche 1 vorgetrieben. Da die Glasampulle 3 an ihrer Rückseite an dem Förderkolben 6 anliegt, wird die Glasampulle 3 mit dem Förderkolben 6 in Richtung des Austragskolbens 7 getrieben. Dabei laufen die Stäbe 9 seitlich an dem Ampullenkopf 11 vorbei. Gleichzeitig verkleinert sich der Innenraum der Aufnahme 2 und die Glasampulle 3 zerbricht, nachdem der Ampullenkopf 11 gegen den Austragskolben 7 gedrückt wird. Dabei bricht zunächst der Ampullenkopf 11 vom Ampullenkörper 10 und wird, geführt von den Stäben 9, in den Ampullenkörper 10 hineingedrückt. Die Monomerflüssigkeit 4 tritt aus der Glasampulle 3 in den Innenraum der Aufnahme 2 aus. Der Austragskolben 7 kann nicht oder nicht weit von der Glasampulle 3 in Richtung des Porenfilters 36 geschoben werden, wenn das Zementpulver 5 trocken ist, also nicht von der Monomerflüssigkeit 4 benetzt ist, da das trockene Zementpulver 5 nicht fließfähig ist und eine Bewegung des Austragskolbens 7 blockiert. Diese Situation ist in der zweiten Abbildung von oben der Figur 4 gezeigt. Überstehende Luft aus der Aufnahme 2 wird durch den Filter 18, die Verbindung 14, den Porenfilter 16, durch die Zwischenräume zwischen den Partikeln des Zementpulvers 5, durch den Porenfilter 36, durch den Schaumstoff 40 und aus den Öffnungen 39 in der Kappe 38 aus der Vorrichtung herausgedrückt.

Bei weiterem Vortreiben des Förderkolbens 6 gleiten die Stäbe 9 in den Ampullenkörper 10 hinein. Gleichzeitig wird der Zwischenraum zwischen dem Förderkolben 6 und dem Austragskolben 7 weiter verkleinert und dabei Luft aus dem Zwischenraum herausgepresst. Wenn die Luft vollständig entwichen ist, wird die freigesetzte Monomerflüssigkeit 4 aus der Aufnahme 2 in den Innenraum der Kartusche 1 und damit in das Zementpulver 5 gepresst. Dabei kann die Monomerflüssigkeit 4 entlang des Hohlzylinders 15 tief in das Zementpulver 5 hineinfließen. Nun trifft die Wandung des Ampullenkörpers 10 auf die Spaltelemente 12 (siehe Figur 4 dritte Abbildung von oben und Figur 8 mittlere Abbildung) und wird dadurch bei weiterem Vortreiben des Förderkolbens 6 und des Ampullenkörpers 10 an den Spaltelementen 12 zersplittert. Die Splitter 52 entstehen dabei von den Spitzen der Stäbe 9 beabstandet. Dabei sammeln sich die Splitter 52 zwischen den Spitzen der Stäbe 9 und der Rückseite des Austragskolbens 7 beziehungsweise dem Filter 18 des Austragskolbens 7. Diese Situation ist in Figur 4, vierte Abbildung von oben und in Figur 8 untere Abbildung dargestellt.

Von der Glasampulle 3 bleiben schließlich nur kleine Splitter 52 zurück, die von dem Filter 18 zurückgehalten werden und in dem röhrenförmigen Behälter verbleiben, der die Kartusche 1 und die Aufnahme 2 bildet. Die Monomerflüssigkeit 4 wird durch den Filter 18, die Verbindung 14 und den Porenfilter 16 in das Zementpulver 5 gepresst und beginnt dort mit dem Zementpulver 5 zu reagieren, so dass sich aus dem Gemisch der Knochenzementteig 54 bildet (siehe Figur 4 unterste Abbildung). Die Menge der Monomerflüssigkeit 4 ist so gewählt, dass das Zementpulver 5 bis in die vorderste Spitze der Kartusche 1, das heißt bis in die Vertiefung 37 im Porenfilter 36 mit der Monomerflüssigkeit 4 benetzt wird. Diese Situation ist in Figur 4, unterste Abbildung gezeigt. Der Porenfilter 36 wird, sobald das Gemisch entstanden ist, von dem auf den Knochenzementteig 54 aufgrund des Drucks auf den Austragskolben 7 wirkenden Druck nach vorne getrieben und komprimiert den Schaumstoff 40. Wenn der Porenfilter 36 nun nach vorne rutscht, so wird er von außen für den Anwender durch die Öffnung 39 in der Kappe 38 sichtbar. Diese Situation ist in Figur 4, unterste Abbildung erkennbar. Hierzu hat der Porenfilter 36 vorzugsweise eine andere Farbe und/oder Helligkeit als der Schaumstoff 40. Beispielsweise kann der Schaumstoff 40 weiß sein und der Porenfilter 36 orange.

In diesem Zustand wird die Kappe 38 mit dem Porenfilter 36 und dem Schaumstoff 40 abgeschraubt und stattdessen eine verlängerte Austragsöffnung in Form eines Applikatorrohrs 66 auf das Austragsrohr 34 aufgeschraubt (siehe Figur 5 und 6). Beim Abschrauben der Kappe 38 wird der vorderste Teil des Knochenzementteigs 54, der sich in der Vertiefung 37 des Porenfilters 38 befindet, mit der Kappe 38 und dem Porenfilter 36 entfernt. Dadurch wird ein potenziell schlechter gemischter Teil des Knochenzementteigs 54 entfernt und somit eine größere Homogenität des verfügbaren Knochenzementteigs 54 erreicht.

Durch weiteres Vortreiben der Stange 44 wird der Förderkolben 6 gegen die Stäbe 9 gepresst, die den minimalen Abstand zwischen dem Förderkolben 6 und dem Austragskolben 7 einstellen, beziehungsweise deren Länge in Längsrichtung der Vorrichtung den Abstand zwischen dem Förderkolben 6 und dem Austragskolben 7 und damit das dazwischen eingeschlossene Volumen bestimmt. Durch noch weiteres Vortreiben der Stange 44 wird der Förderkolben 6, die Scherben 52 und der davor angeordnete und durch die Stäbe 9 beabstandete Austragskolben 7 angetrieben. Über das Applikatorrohr 66 wird dann der Knochenzementteig 54 aus der Kartusche 1 ausgetragen. Dazu wird der Austragskolben 7 mit der Stange 44 in Richtung des Austragsrohrs 34 vorgetrieben (siehe Figur 5). Der Knochenzementteig 54 aus dem Inneren der Kartusche 1 wird durch das Austragsrohr 34 und das Applikatorrohr 66 ausgetrieben und kann dort appliziert werden oder auch zur weiteren Verarbeitung verwendet werden.

Schließlich trifft der Hohlzylinder 15 auf vordere Innenseite des Innenraums der Kartusche 1. Der Hohlzylinder 15 schließt dabei ein Volumen des Knochenzementteigs 54 ein, welches dem Austragskolben 7 am nächsten liegt. Dieser Knochenzementteig 54 wird in der Vorrichtung zurückgehalten. Durch die am Ende des Auspressvorgangs auftretenden Kräfte im Inneren der Vorrichtung kann es zu einer Nachverdichtung kommen und dabei zu einer geringfügigen Veränderung der Konsistenz des Knochenzementteigs 54, der deswegen in der Kartusche 1 zurückgehalten wird. Durch den Hohlzylinder 15 entsteht im Innenraum der Kartusche 1 ein Totvolumen, das nicht aus der Kartusche 1 durch die Austragsöffnung und das Austragsrohr 34 ausgetrieben werden kann. In diesem Totvolumen befindet sich nun der Teil des Knochenzementteigs 54, der gegebenenfalls einen zu großen Anteil an Monomerflüssigkeit 4 enthält. Durch diesen Aufbau wird sichergestellt, dass kein Knochenzementteig 54 mit einer sich ändernden Konsistenz aufgrund sich ändernder Zusammensetzung mit der Vorrichtung appliziert werden kann.

Die Öffnungen 39 dienen auch als visuelle Marker anhand derer festgestellt werden kann, wann die Vorrichtung einsatzbereit ist. Wenn der Porenfilter 36 aufgrund des Drucks des Knochenzementteigs 54 nach vorne gedrückt wird und dabei das Styropor 40 in der Kappe 38 zusammenpresst, wird der Porenfilter 36 durch die Öffnungen 39 sichtbar. Dadurch kann der Anwender erkennen, dass der Knochenzementteig 54 fertig gemischt in der Kartusche 1 vorliegt und damit einsatzbereit ist. Der Anwender kann zu diesem Zeitpunkt die Kappe 38 mit dem Porenfilter 36 abschrauben und das Applikatorrohr 66 auf das Austragsrohr 34 aufschrauben. Anschließend kann der Austragskolben 7 über den Förderkolben 6 mit der Stange 44 angetrieben werden und dadurch der Knochenzementteig 54 aus der Kartusche 1 durch das Applikatorrohr 66 ausgetrieben werden.

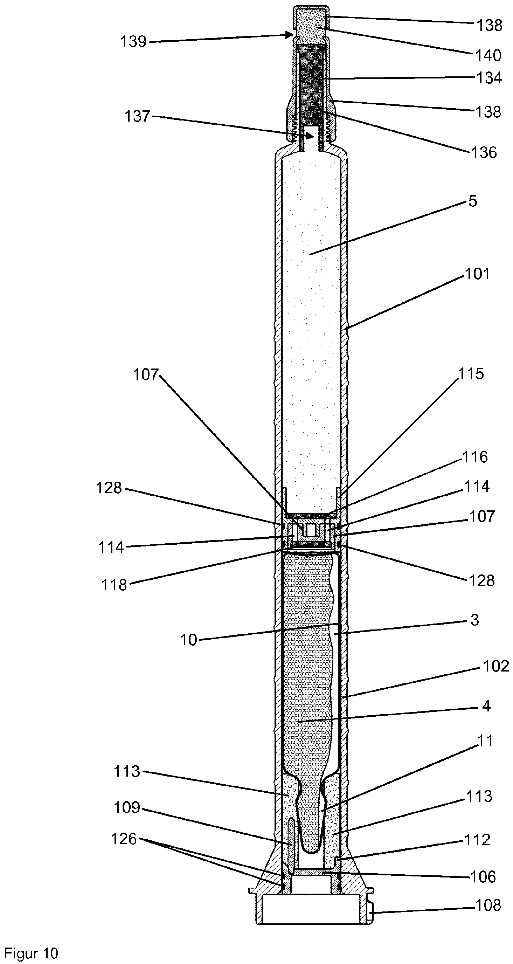

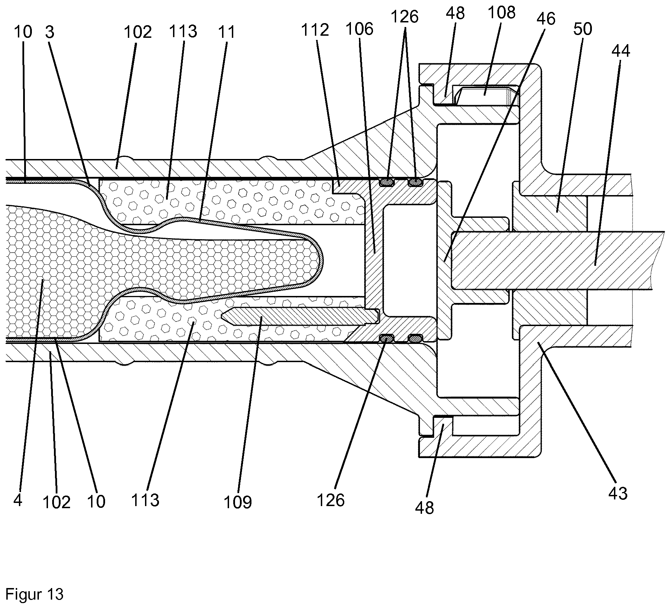

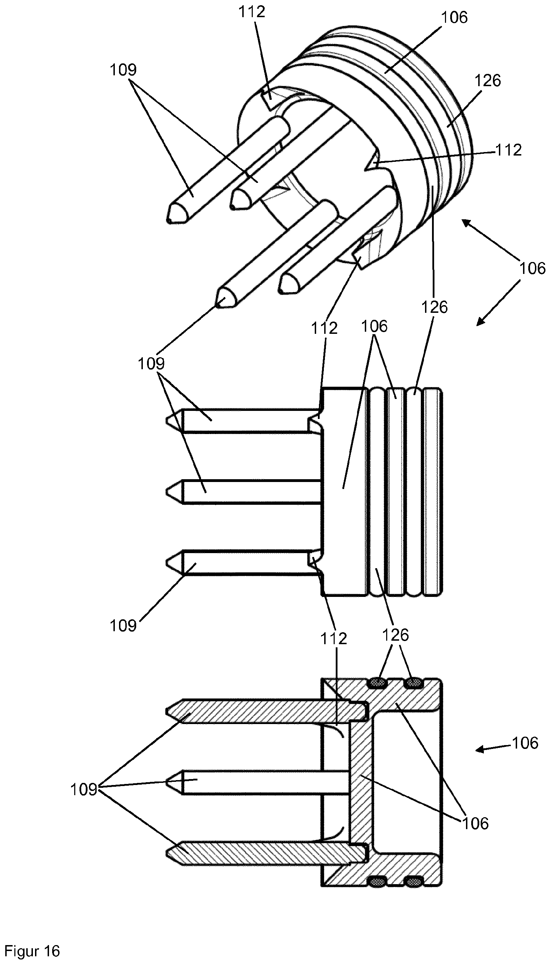

In den Figuren 10 bis 16 sind Abbildungen einer zweiten erfindungsgemäßen Vorrichtung zum Lagern und Mischen eines Knochenzementteigs gezeigt. Die Figuren 10 bis 12 zeigen verschiedene schematische Gesamtansichten der zweiten beispielhaften erfindungsgemäßen Vorrichtung. Die Figuren 13 bis 15 zeigen Ausschnittvergrößerungen von schematischen Querschnittansichten als Detailansichten durch verschiedene Bereiche der zweiten erfindungsgemäßen Vorrichtung und Figur 16 zeigt drei schematische Detailansichten eines Förderkolbens der zweiten erfindungsgemäßen Vorrichtung. Die zweite erfindungsgemäße Vorrichtung entspricht in ihrem äußeren Aufbau sehr weitgehend und bezüglich der Ausgangskomponenten vollständig dem Aufbau der ersten erfindungsgemäßen Vorrichtung.

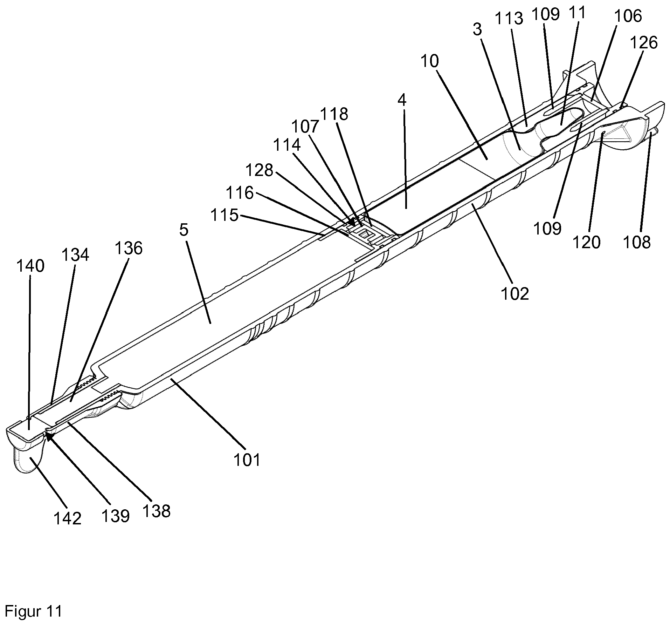

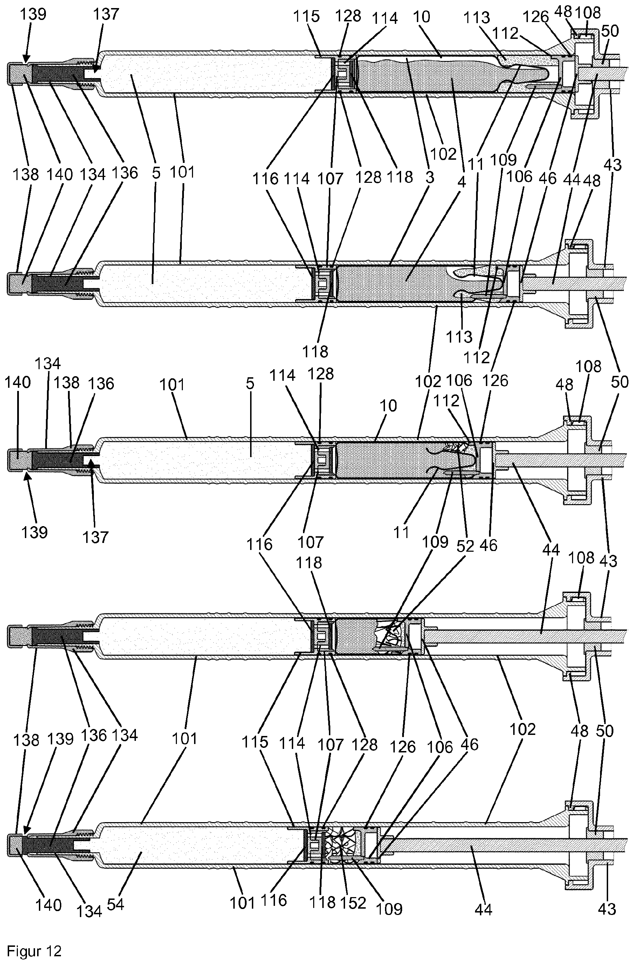

Die zweite erfindungsgemäße Vorrichtung besteht im Wesentlichen aus einem röhrenförmigen Behälter aus Kunststoff, der als vorderen Teil (in den Figur 10 oben, in Figur 11 links unten und in den Figuren 12 bis 15 links) eine Kartusche 101 mit einem zylindrischen Innenraum bildet und der als hinteren Teil eine Aufnahme 102 mit einem zylindrischen Innenraum für eine Glasampulle 3 (oder eine Kunststoffampulle 3) als Behälter für eine Monomerflüssigkeit 4 bildet. Die Rückseite der Vorrichtung ist in den Figur 10 unten, in Figur 11 rechts oben und in Figur 12 rechts gezeigt. Die Röhrenform des Behälters ist besonders gut in den Querschnittansichten der Figuren 10 und 11 zu erkennen. Sowohl der Innenraum der Kartusche 101 als auch der Innenraum der Aufnahme 102 sind zylindrisch mit kreisförmiger Grundfläche. Dabei sind die Durchmesser des Innenraums der Kartusche 101 und der Durchmesser des Innenraums der Aufnahme 102 gleich groß und fluchten miteinander. Der Behälter mit der Aufnahme 102 und der Kartusche 101 wird vorzugsweise mit Hilfe einer Spritzgusstechnik aus Kunststoff hergestellt. Die Aufnahme 102 weist also einen zylindrischen Innenraum auf, in den die Glasampulle 3 eingesteckt ist. In der Glasampulle 3 befindet sich die Monomerflüssigkeit 4. In den Innenraum der Kartusche 101 ist ein Zementpulver 5 eingefüllt oder vorzugsweise eingepresst. Die Monomerflüssigkeit 4 und das Zementpulver 5 bilden die Ausgangskomponenten für einen PMMA-Knochenzement, der mit der Vorrichtung hergestellt werden kann. Aufgrund der Glasampulle 3 kann die Monomerflüssigkeit 4 sehr lange in der Aufnahme 102 und dadurch in der Vorrichtung gelagert werden. Das Zementpulver 5 kann ebenfalls über längere Zeiträume in der Vorrichtung gelagert werden. Die Vorrichtung ist damit zum Lagern der Monomerflüssigkeit 4 und des Zementpulvers 5 als Ausgangskomponenten eines Knochenzementteigs des PMMA-Knochenzements geeignet. Die Vorrichtung ist aber auch zum Mischen des Knochenzementteigs aus den Ausgangskomponenten und zum Austragen des gemischten Knochenzementteigs geeignet und vorgesehen.

In der Aufnahme 102 ist ein im zylindrischen Innenraum der Aufnahme 102 in Längsrichtung beweglicher Förderkolben 106 aus Kunststoff angeordnet. Der Förderkolben 106 ist im Bereich der Rückseite der Aufnahme 102 angeordnet. Die Glasampulle 3 kann mit dem Förderkolben 106 in der Aufnahme 102 zusammengedrückt und dabei zersplittert werden, indem der Förderkolben 106 in Richtung der Vorderseite, also in Richtung der Kartusche 101 gedrückt wird. Der Förderkolben 106 weist an der Vorderseite Abstreifer auf, mit denen Splitter 52 der Glasampulle 3 von der Innenwand der Aufnahme 102 abgestreift werden. Die Abstreifer liegen hierzu seitlich an der Innenwand des Innenraums der Aufnahme an.

In dem Innenraum der Kartusche 1 ist in dessen Rückseite (in Figur 10 Richtung unten, in Figur 11 nach rechts oben, in den Figuren 12 bis 15 Richtung rechts) ein Austragskolben 107 aus Kunststoff angeordnet, der in den Abbildungen nach Figur 16 im Detail dargestellt ist. An der Rückseite der Aufnahme 102 ist ein Befestigungsmittel 108 vorgesehen, mit dem die Aufnahme 102 an einer Auspressvorrichtung 43 (in den Figuren 10 und 11 nicht zu sehen, siehe aber Figur 12) verbunden werden kann. Das Befestigungsmittel 108 ist vorzugsweise zur Bildung eines Bajonettverschlusses geeignet und vorgesehen. Dadurch kann der Förderkolben 106, der von der Rückseite der Aufnahme 102 aus frei zugänglich ist, mit der Auspressvorrichtung 43 in Richtung der Vorderseite der Kartusche 101 vorgetrieben werden.

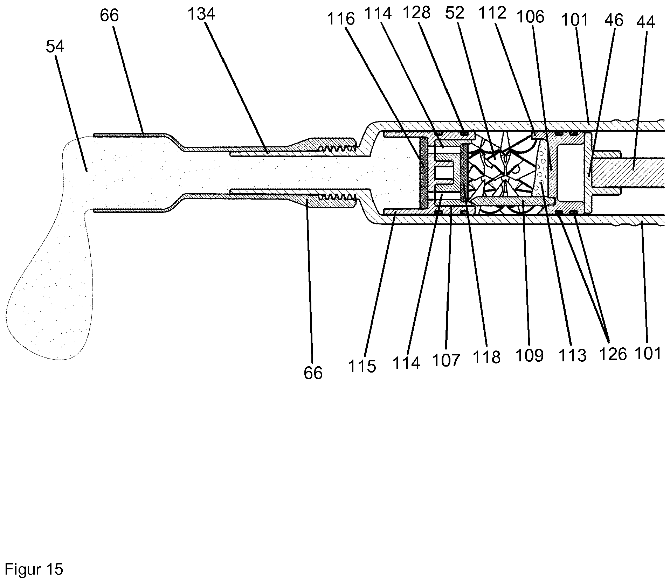

Der Förderkolben 106 weist an seiner Vorderseite vier Stäbe 109 als Abstandhalter auf, die den Abstand zwischen dem Austragskolben 107 und dem Förderkolben 106 bestimmen, wenn der Förderkolben 106 vollständig in Richtung des Austragskolbens 107 gedrückt ist (siehe Figur 15). Die Stäbe 109 erstrecken sich mindestens 10 mm von der Vorderseite des Förderkolbens 106 in die Aufnahme 102 hinein. Die Stäbe 109 sind rotationssymmetrisch (beispielsweise zylindrisch), können aber auch einen eckigen Querschnitt aufweisen. Die Stäbe 109 sind in Richtung des Austragskolbens 107 zulaufend geformt, wobei die Spitzen ein stumpfes Ende aufweisen (siehe Figur 16). Durch die spitz zulaufende Spitze der Stäbe 109 können zwischen den Spitzen der Stäbe 109 und dem Austragskolben 107 entstehende Splitter 52 beim Zerdrücken der Glasampulle 3 durch die Bewegung des Förderkolbens 106 leichter seitlich an den Stäben 109 vorbeigedrückt werden. Durch das stumpfe Ende der Stäbe 109 wird verhindert, dass sich die Stäbe 109 in den Austragskolben 107 eindrücken oder die Stäbe 109 an der Spitze verformt werden und dadurch sich die Länge der Stäbe 109 verändert und dadurch der Abstand zwischen dem Austragskolben 107 und dem Förderkolben 106 und damit der Raum dazwischen variiert und weniger gut vorherbestimmbar wird. Dadurch ist die in dem Zwischenraum zwischen dem Austragskolben 107 und dem Förderkolben 106 verbleibende Menge der Monomerflüssigkeit 4 nach dem Auspressen (siehe Figur 15) und damit die in das Zementpulver 5 eingepresste Menge der Monomerflüssigkeit 4 sehr genau bekannt und vorhersehbar. Dadurch ist die Konsistenz des erzeugten Knochenzementteigs 54 sehr genau einstellbar und reproduzierbar.

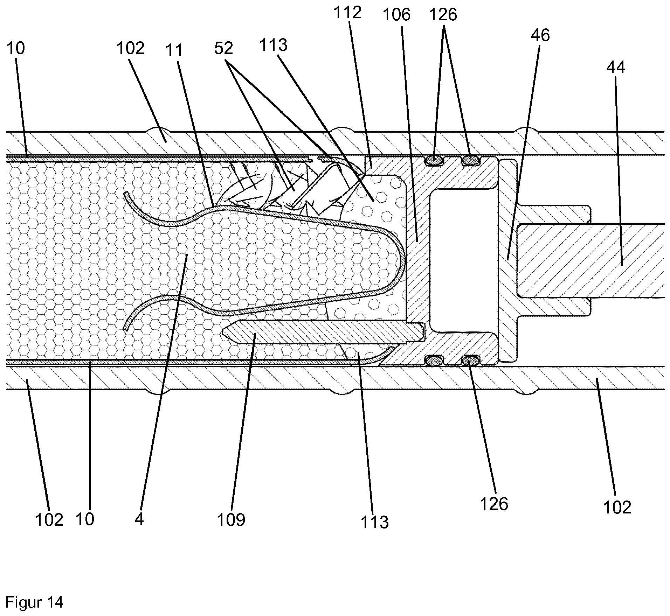

Die Glasampulle 3 weist einen Ampullenkörper 10 und einen Ampullenkopf 11 auf, die über einen dünnen Hals miteinander verbunden sind. Die Glasampulle 3 kann sehr einfach durch Abbrechen des Ampullenkopfs 11 vom Ampullenkörper 10 geöffnet werden. Die Stäbe 109 verlaufen seitlich vom Ampullenkopf 11 und umgeben diesen (siehe Figuren 10, 11 und 12), so dass bei einer Bewegung der Glasampulle 3 durch Vortreiben des Förderkolbens 106 die Stäbe 109 seitlich neben dem Ampullenkopf 11 angeordnet bleiben und in die Schultern der Glasampulle 3 in den Ampullenkörper 10 hineingetrieben werden (siehe Figuren 12 und 14). Die Glasampulle 3 ist bei der zweiten beispielhaften Vorrichtung umgekehrt in der Aufnahme 102 angeordnet im Vergleich zu der Glasampulle 3 bei der ersten beispielhaften Vorrichtung nach den Figuren 1 bis 9. Als grundlegendes Prinzip ist nämlich der Ampullenkopf 11 immer in Richtung der Stäbe 9, 109 ausgerichtet, unabhängig davon ob diese an dem Austragskolben 7 oder am Förderkolben 106 befestigt sind.

Die Stäbe 109 sind elastisch verformbar und aus einem Kunststoff gefertigt. Durch die elastische Verformbarkeit können Bruchstücke der Glasampulle 3 leichter an den Stäben 109 vorbeigeleitet werden. Die Stäbe 109 sind von der Innenwand der Aufnahme 102 zumindest so weit beabstandet, dass die Wandung des Ampullenkörpers 10 zwischen die Stäbe 109 und die Innenwand der Aufnahme 102 passt. Dadurch verlaufen die Stäbe 109 beim Vortreiben des Förderkolbens 106 im Inneren des Ampullenkörpers 10.

Es kann vorgesehen sein, dass die Stäbe 109 über wenigstens einen gemeinsamen Ring (nicht gezeigt) miteinander verbunden sind. Der wenigstens eine Ring ist parallel zur Vorderseite des Förderkolbens 106 angeordnet. Beispielsweise können zwei Ringe vorgesehen sein, wobei ein erster Ring mit einem Drittel der Länge der Stäbe 109 von der Vorderseite des Förderkolbens 106 beabstandet ist und ein zweiter Ring mit zwei Dritteln der Länge der Stäbe 109 von der Vorderseite des Förderkolbens 106 beabstandet ist. Hierdurch werden die Stäbe 109 stabilisiert und können weniger leicht abknicken. Je länger die Stäbe 109 sind, desto vorteilhafter ist eine Stabilisierung mit wenigstens einem Ring. Der Innendurchmesser des wenigstens einen Rings muss dabei groß genug sein, um den Ampullenkopf 11 aufnehmen zu können.

An der Vorderseite des Förderkolbens 106 sind vier keilförmige Spaltelemente 112 angeordnet, die zum Spalten oder Brechen des Ampullenkörpers 10 der Glasampulle 3 bei Vortreiben des Förderkolbens 106 vorgesehen sind. Dazu verlaufen die Kanten der Spaltelemente 112 radial nach außen und sind außen am Förderkolben 106 angeordnet, so dass die Kanten der Spaltelemente 112 durch die komplette Wandung des Ampullenkörpers 10 laufen können und diesen dabei zersplittern. Die Spitzen der Stäbe 109 sind von den Spaltelementen 112 in Längsrichtung der Vorrichtung beabstandet, so dass die Brechfront, an der der Ampullenkörper 10 zersplittert wird, von den Spitzen der Stäbe 109 beabstandet ist. Hierdurch kann vermieden werden, dass viele Splitter 52 zwischen den Spitzen der Stäbe 109 und dem Austragskolben 107 entstehen, die zwischen den Spitzen der Stäbe 109 und dem Austragskolben 107 eingeklemmt werden könnten und dadurch das minimale Volumen zwischen dem Förderkolben 106 und dem Austragskolben 107 und damit die in das Zementpulver 5 eingepresste Menge der Monomerflüssigkeit 4 beeinflussen können.

Zur stabilen Lagerung der Glasampulle 3 ist eine Lagerung 113 in Form einer Hülse aus Schaumstoff als Stoßsicherung vorgesehen. Die hülsenförmige Lagerung 113 umgibt den Ampullenkopf 11 und ist zwischen den Stäben 109 und der Innenwand der Aufnahme 102 eingesteckt. Die Lagerung 113 ist für die Monomerflüssigkeit 4 durchlässig und aus einem Kunststoff gefertigt.

Die Kartusche 101 und die Aufnahme 102 sind einteilig als gemeinsames Kunststoffteil ausgeführt. Die Aufnahme 102 und die Kartusche 101 sind für die Monomerflüssigkeit 4 flüssigkeitsdurchlässig miteinander über eine Verbindung 114 im Austragskolben 107 verbunden. An der Vorderseite des Austragskolbens 107 ist ein Hohlzylinder 115 angeordnet. Die Verbindung 114 durch den Austragskolben 107 mündet durch einen für das Zementpulver 5 undurchlässigen aber für die Monomerflüssigkeit 4 durchlässigen Porenfilter 116 in den Innenraum der Kartusche 101.

In der Einmündung zur Verbindung 114 ist in dem Austragskolben 107 ein Filter 118 angeordnet, mit dem die Splitter 52 der Glasampulle 3 zurückgehalten werden können. Statt dem Filter 118 oder zusätzlich zum Filter 118 kann auch ein Sieb vorgesehen sein.

Mehrere Belüftungsöffnungen 120 sind in der Wandung der Aufnahme 102 vorgesehen, durch die der Innenraum der Aufnahme 102 mit Hilfe eines sterilisierenden Gases wie Ethylenoxid sterilisiert werden kann. Die Belüftungsöffnungen 120 sind unmittelbar benachbart zum Förderkolben 106 angeordnet, so dass der Förderkolben 106 sich unmittelbar vor die Belüftungsöffnungen 120 schiebt und somit die Belüftungsöffnungen 120 unmittelbar verschließt, wenn der Förderkolben 106 in Richtung der Kartusche 101 vorgetrieben wird. Dadurch wird verhindert, dass Monomerflüssigkeit 4 durch die Belüftungsöffnungen 120 austreten kann, wenn die Glasampulle 3 in der Aufnahme 102 geöffnet wurde.

Der zylindrische Förderkolben 106 hat einen zur Zylindergeometrie des Innenraums der Aufnahme 102 passenden Außenumfang und ist über zwei umlaufende Dichtungen 126 gegen die Innenwand der Aufnahme 102 flüssigkeitsdicht abgedichtet. Ebenso ist der Austragskolben 107 über zwei umlaufende Dichtungen 128 gegen die Innenwand der Kartusche 101 flüssigkeitsdicht abgedichtet. Diese Dichtungen 126, 128 dienen dazu, einen Austritt von Monomerflüssigkeit 4 oder von Knochenzementteig 54 zu verhindern, um eine Kontamination der Umgebung (des OP-Saals und des Anwenders) zu verhindern. Die Dichtungen 126, 128 können hierzu aus Gummi bestehen.

Der Innenraum der Kartusche 101 mündet an der Vorderseite in ein Austragsrohr 134, das eine vordere Austragsöffnung der Kartusche 101 begrenzt. Das Austragsrohr 134 weist an seiner Basis ein Außengewinde auf. Im Inneren der Austragsrohrs 134 ist ein Porenfilter 136 als Verschluss für die Kartusche 101 angeordnet. Der Porenfilter 136 ist für das Zementpulver 5 undurchlässig aber für Gase durchlässig. In der Rückseite des Porenfilters 136 ist eine Vertiefung 137 vorgesehen. Das Zementpulver 5 ist auch in der Vertiefung 137 enthalten. An dem Außengewinde des Austragsrohrs 134 ist eine Kappe 138 befestigt, wobei der vordere Teil der Kappe 138 mit einem Styropor oder Schaumstoff 140 gefüllt ist. An der Kappe 138 sind zwei Flügel 142 vorgesehen, so dass die Kappe 138 nach Art einer Flügelschraube bequem von dem Austragsrohr 134 abgeschraubt werden kann. Die Kappe 138 weist seitliche Öffnungen 139 auf. Durch diesen Aufbau kann das Innere der Kartusche 101 und das Zementpulver 5 mit Hilfe von Ethylenoxid sterilisiert werden, da die Öffnungen 139 in der Kappe 138, das Styropor oder der Schaumstoff 140, der Porenfilter 136 und die Zwischenräume zwischen den Pulverpartikeln des Zementpulvers 5 luftdurchlässig sind. Gleichzeitig kann Luft aus der Aufnahme 102 durch das Zementpulver 5, den Porenfilter 136, das Styropor oder der Schaumstoff 140 und die Öffnungen 139 in der Kappe 138 herausgepresst werden, wenn der Förderkolben 106 in Richtung der Aufnahme 101 gepresst wird. Die Kappe 138 bildet zusammen mit dem Styropor oder Schaumstoff 140 und mit dem Porenfilter 136 einen Verschluss für die Austragsöffnung der Kartusche 101 beziehungsweise für das Austragsrohr 134.

Das Zementpulver 5 ist in der Kartusche 101 eingeschlossen, da alle Öffnungen 139 und Verbindungen 114 mit Hilfe der Porenfilter 116, 136 für das Zementpulver 5 undurchlässig verschlossen sind. Der Inhalt der Kartusche 101 kann dabei durch Evakuieren und Spülen mit Ethylenoxid sterilisiert werden. Dadurch ist die Vorrichtung auch zum langfristigen Lagern des Zementpulvers 5 geeignet.

Figur 12 zeigt fünf schematische Querschnittansichten der zweiten erfindungsgemäßen Vorrichtung nach den Figuren 10 bis 16 übereinander zur Verdeutlichung des Ablaufs eines erfindungsgemäßen Verfahrens. Als letzter Schritt des Verfahrens wird schließlich ein Zustand erreicht, der in Figur 15 als Ausschnittvergrößerung dargestellt ist. Dazu zeigt Figur 13 eine Ausschnittvergrößerung der obersten Abbildung der Figur 12 und Figur 14 eine Ausschnittvergrößerung der dritten Abbildung von oben der Figur 12.

Zu Beginn des Verfahrens liegt die Vorrichtung im Ausgangszustand vor, so wie sie auch in den Figuren 10 und 11 gezeigt ist. In diesem Zustand wird die Vorrichtung in eine erfindungsgemäße Auspressvorrichtung 43 eingesetzt, die weitgehend einer herkömmlichen Kartuschenpistole entspricht. Diese Situation ist in der obersten Abbildung von Figur 12 gezeigt. Die Auspressvorrichtung 43 umfasst eine linear vortreibbare Stange 44. Von der Auspressvorrichtung 43 ist nur der vordere Teil dargestellt. Die Auspressvorrichtung 43 gleicht der Auspressvorrichtung 43, die in der Beschreibung des ersten Ausführungsbeispiels nach den Figuren 1 bis 9 beschrieben wurde, und umfasst auch einen Griff und einen Kipphebel (in den Abbildungen nicht zu sehen) zum manuellen Antreiben der Stange 44 der Auspressvorrichtung 43, wie bei herkömmlichen manuell angetriebenen Auspressvorrichtungen 43 auch. Die Vorrichtung wird mit dem Befestigungsmittel 108 an der Auspressvorrichtung 43 befestigt (siehe oberste Abbildung in Figur 12 und Figur 13). An der Spitze der Stange 44 ist ein flacher Teller 46 zum Antreiben des Förderkolbens 106 vorgesehen. Die Stange 44 drückt mit dem Teller 46 auf den Förderkolben 106, wenn die Stange 44 von der Auspressvorrichtung 43 in die Aufnahme 102 hineingedrückt wird. Die Auspressvorrichtung 43 ist hierzu über ein Gegenbefestigungsmittel 48 an die Rückseite der Aufnahme 102 angeschlossen, so dass der Teller 46 beim Vortreiben der Stange 44 auf den Förderkolben 106 drückt und diesen in Richtung der Kartusche 101 vortreibt. Hierzu ist die Stange 44 gegen ein Lager 50 und darüber gegen das Gegenbefestigungsmittel 48 und damit gegen die Aufnahme 102 linear beweglich gelagert.

Die Auspressvorrichtung 43 wird bedient und dabei die Stange 44 und mit der Stange 44 der Förderkolben 106 in Richtung der Kartusche 101 vorgetrieben. Da die Glasampulle 3 an ihrer Rückseite an dem Förderkolben 106 anliegt, wird die Glasampulle 3 mit dem Förderkolben 106 in Richtung des Austragskolbens 107 getrieben. Dabei bleiben die Stäbe 109 seitlich neben dem Ampullenkopf 11 angeordnet. Gleichzeitig verkleinert sich der Innenraum der Aufnahme 102 und die Glasampulle 3 zerbricht, nachdem der Boden der Glasampulle 3 gegen den Austragskolben 107 gedrückt wird. Dabei bricht zunächst der Ampullenkopf 11 vom Ampullenkörper 10 und wird, geführt von den Stäben 109, in den Ampullenkörper 10 hineingedrückt. Die Monomerflüssigkeit 4 tritt aus der Glasampulle 3 in den Innenraum der Aufnahme 102 aus. Der Austragskolben 107 kann nicht oder nicht weit von der Glasampulle 3 in Richtung des Porenfilters 136 geschoben werden, wenn das Zementpulver 5 trocken ist, also nicht von der Monomerflüssigkeit 4 benetzt ist, da das trockene Zementpulver 5 nicht fließfähig ist und eine Bewegung des Austragskolbens 107 blockiert. Diese Situation ist in der zweiten Abbildung von oben der Figur 12 gezeigt. Überstehende Luft aus der Aufnahme 102 wird durch den Filter 118, die Verbindung 114, den Porenfilter 116, durch die Zwischenräume zwischen den Partikeln des Zementpulvers 5, durch den Porenfilter 136, durch den Schaumstoff 140 und aus den Öffnungen 139 in der Kappe 138 aus der Vorrichtung herausgedrückt.

Bei weiterem Vortreiben des Förderkolbens 106 gleiten die Stäbe 109 in den Ampullenkörper 10 hinein. Gleichzeitig wird der Zwischenraum zwischen dem Förderkolben 106 und dem Austragskolben 107 weiter verkleinert und dabei Luft aus dem Zwischenraum herausgepresst. Wenn die Luft vollständig entwichen ist, wird die freigesetzte Monomerflüssigkeit 4 aus der Aufnahme 102 in den Innenraum der Kartusche 101 und damit in das Zementpulver 5 gepresst. Dabei kann die Monomerflüssigkeit 104 entlang des Hohlzylinders 115 tief in das Zementpulver 5 hineinfließen. Nun trifft die Wandung des Ampullenkörpers 10 auf die Spaltelemente 112 (siehe Figur 12 dritte Abbildung von oben und Figur 14) und wird dadurch bei weiterem Vortreiben des Förderkolbens 106 und des Ampullenkörpers 10 an den Spaltelementen 112 zersplittert. Die Splitter 52 entstehen dabei von den Spitzen der Stäbe 109 beabstandet. Dabei sammeln sich die Splitter 52 zwischen den Spitzen der Stäbe 109 und der Vorderseite des Förderkolbens 106. Diese Situation ist in Figur 12, vierte Abbildung von oben dargestellt.

Von der Glasampulle 3 bleiben schließlich nur kleine Splitter 52 zurück, die von dem Filter 118 zurückgehalten werden und in dem röhrenförmigen Behälter verbleiben, der die Kartusche 101 und die Aufnahme 102 bildet. Die Monomerflüssigkeit 4 wird durch den Filter 118, die Verbindung 114 und den Porenfilter 116 in das Zementpulver 5 gepresst und beginnt dort mit dem Zementpulver 5 zu reagieren, so dass sich aus dem Gemisch der Knochenzementteig 54 bildet (siehe Figur 12 unterste Abbildung). Die Menge der Monomerflüssigkeit 4 ist so gewählt, dass das Zementpulver 5 bis in die vorderste Spitze der Kartusche 101, das heißt bis in die Vertiefung 137 im Porenfilter 136 mit der Monomerflüssigkeit 4 benetzt wird. Diese Situation ist in Figur 12, unterste Abbildung gezeigt. Der Porenfilter 136 wird, sobald das Gemisch entstanden ist, von dem auf den Knochenzementteig 54 aufgrund des Drucks auf den Austragskolben 107 wirkenden Druck nach vorne getrieben und komprimiert den Schaumstoff 140. Wenn der Porenfilter 136 nun nach vorne rutscht, so wird er von außen für den Anwender durch die Öffnung 139 in der Kappe 138 sichtbar. Diese Situation ist in Figur 12, unterste Abbildung erkennbar. Hierzu hat der Porenfilter 136 vorzugsweise eine andere Farbe und/oder Helligkeit als der Schaumstoff 140. Beispielsweise kann der Schaumstoff 140 blau sein und der Porenfilter 136 gelb.

In diesem Zustand wird die Kappe 138 mit dem Porenfilter 136 und dem Schaumstoff 140 abgeschraubt und stattdessen eine verlängerte Austragsöffnung in Form eines Applikatorrohrs 66 auf das Austragsrohr 134 aufgeschraubt (siehe Figur 14). Beim Abschrauben der Kappe 138 wird der vorderste Teil des Knochenzementteigs 54, der sich in der Vertiefung 137 des Porenfilters 138 befindet, mit der Kappe 138 und dem Porenfilter 136 entfernt. Dadurch wird ein potenziell schlechter gemischter Teil des Knochenzementteigs 54 entfernt und somit eine größere Homogenität des verfügbaren Knochenzementteigs 54 erreicht.

Durch weiteres Vortreiben der Stange 44 wird der Förderkolben 106 mit den Stäben 109 gegen den Austragskolben 107 gepresst, die den minimalen Abstand zwischen dem Förderkolben 106 und dem Austragskolben 107 einstellen, beziehungsweise deren Länge in Längsrichtung der Vorrichtung den Abstand zwischen dem Förderkolben 106 und dem Austragskolben 107 und damit das dazwischen eingeschlossene Volumen bestimmt. Durch noch weiteres Vortreiben der Stange 44 wird der Förderkolben 106, die Scherben 52 und der davor angeordnete und durch die Stäbe 109 beabstandete Austragskolben 107 angetrieben. Über das Applikatorrohr 66 wird dann der Knochenzementteig 54 aus der Kartusche 101 ausgetragen. Dazu wird der Austragskolben 107 mit der Stange 44 in Richtung des Austragsrohrs 134 vorgetrieben (siehe Figur 15). Der Knochenzementteig 54 aus dem Inneren der Kartusche 101 wird durch das Austragsrohr 134 und das Applikatorrohr 66 ausgetrieben und kann dort appliziert werden oder auch zur weiteren Verarbeitung verwendet werden.

Schließlich trifft der Hohlzylinder 115 auf vordere Innenseite des Innenraums der Kartusche 101. Der Hohlzylinder 115 schließt dabei ein Volumen des Knochenzementteigs 54 ein, welches dem Austragskolben 107 am nächsten liegt. Dieser Knochenzementteig 54 wird in der Vorrichtung zurückgehalten. Durch die am Ende des Auspressvorgangs auftretenden Kräfte im Inneren der Vorrichtung kann es zu einer Nachverdichtung kommen und dabei zu einer geringfügigen Veränderung der Konsistenz des Knochenzementteigs 54, der deswegen in der Kartusche 101 zurückgehalten wird. Durch den Hohlzylinder 115 entsteht im Innenraum der Kartusche 101 ein Totvolumen, das nicht aus der Kartusche 101 durch die Austragsöffnung und das Austragsrohr 134 ausgetrieben werden kann. In diesem Totvolumen befindet sich nun der Teil des Knochenzementteigs 54, der gegebenenfalls einen zu großen Anteil an Monomerflüssigkeit 4 enthält. Durch diesen Aufbau wird sichergestellt, dass kein Knochenzementteig 54 mit einer sich ändernden Konsistenz aufgrund sich ändernder Zusammensetzung mit der Vorrichtung appliziert werden kann.

Die Öffnungen 139 dienen auch als visuelle Marker anhand derer festgestellt werden kann, wann die Vorrichtung einsatzbereit ist. Wenn der Porenfilter 136 aufgrund des Drucks des Knochenzementteigs 54 nach vorne gedrückt wird und dabei das Styropor 140 in der Kappe 138 zusammenpresst, wird der Porenfilter 136 durch die Öffnungen 139 sichtbar. Dadurch kann der Anwender erkennen, dass der Knochenzementteig 54 fertig gemischt in der Kartusche 101 vorliegt und damit einsatzbereit ist. Der Anwender kann zu diesem Zeitpunkt die Kappe 138 mit dem Porenfilter 136 abschrauben und das Applikatorrohr 66 auf das Austragsrohr 134 aufschrauben. Anschließend kann der Austragskolben 107 über den Förderkolben 106 mit der Stange 44 angetrieben werden und dadurch der Knochenzementteig 54 aus der Kartusche 101 durch das Applikatorrohr 66 ausgetrieben werden.

Gemäß einer weiteren alternativen erfindungsgemäßen Ausführungsform, die den vorherigen Ausführungsformen weitgehend entspricht, kann vorgesehen sein, dass Stäbe als Abstandhalter über einen zum Austragskolben und zum Förderkolben separaten Ring miteinander verbunden sind. Die Stäbe müssen dann auch nicht mit dem Förderkolben oder dem Austragskolben verbunden sein, sondern können als lose und separate Abstandhalter zwischen dem Förderkolben und der Glasampulle 3 und/oder zwischen dem Austragskolben und der Glasampulle 3 in der Aufnahme angeordnet sein. Durch die Ringform und durch das Aufstecken des losen Abstandhalters auf den Ampullenkopf 11 und/oder durch Einsetzen des losen Abstandhalters in eine hülsenförmige Lagerung, wie die hülsenförmigen Lagerungen 13, 113 nach dem ersten und dem zweiten Ausführungsbeispiel, sind solche Abstandhalter auch automatisch von der Innenwand der Aufnahme zumindest so weit beabstandet, wie die Dicke der Wandung des Ampullenkörpers 10.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 101: Kartusche
- 2, 102: Aufnahme
- 3: Ampulle
- 4: Monomerflüssigkeit
- 5: Zementpulver
- 6, 106: Förderkolben
- 7, 107: Austragskolben
- 8, 108: Befestigungsmittel / Bajonettverschluss
- 9, 109: Abstandhalter / Stab
- 10: Ampullenkörper
- 11: Ampullenkopf
- 12, 112: Spaltelement / Keil
- 13, 113: Lagerung / Schaumstoff
- 14, 114: Verbindung
- 15, 115: Hohlzylinder
- 16, 116: Porenfilter
- 18, 118: Filter
- 20, 120: Belüftungsöffnung
- 26, 126: Dichtung
- 28, 128: Dichtung
- 34, 134: Austragsrohr
- 36, 136: Porenfilter
- 37, 137: Vertiefung
- 38, 138: Kappe
- 39, 139: Öffnung
- 40, 140: Schaumstoff
- 42, 142: Flügel
- 43: Auspressvorrichtung / Kartuschenpistole
- 44: Stange
- 46: Teller
- 48: Gegenbefestigungsmittel / Bajonettverschluss
- 50: Lagerung
- 52: Splitter
- 54: Knochenzementteig / Gemisch
- 66: Applikatorrohr

## Patentansprüche

1. Vorrichtung zum Herstellen eines Knochenzementteigs (54) aus einer Monomerflüssigkeit (4) und einem Zementpulver (5) als Ausgangskomponenten des Knochenzementteigs (54) und zum Austragen des gemischten Knochenzementteigs (54), die Vorrichtung aufweisend
eine Kartusche (1, 101) mit einem zylindrischen Innenraum, wobei im Innenraum der Kartusche (1, 101) ein in Richtung einer Vorderseite der Kartusche (1, 101) beweglicher Austragskolben (7, 107) in einer Rückseite der Kartusche (1, 101) angeordnet ist,
eine Aufnahme (2, 102), die sich entlang einer Längsrichtung erstreckt, wobei eine Vorderseite der Aufnahme (2, 102) mit der Rückseite der Kartusche (1, 101) verbunden ist,
einen Förderkolben (6, 106), der in der Aufnahme (2, 102) angeordnet ist, wobei der Förderkolben (6, 106) in Längsrichtung der Aufnahme (2, 102) in Richtung zur Vorderseite der Aufnahme (2, 102) hin beweglich in der Aufnahme (2, 102) gelagert ist,
eine aufbrechbare Ampulle (3) enthaltend die Monomerflüssigkeit (4), wobei die Ampulle (3) zwischen dem Förderkolben (6, 106) und dem Austragskolben (7, 107) in der Aufnahme (2, 102) angeordnet ist und wobei die Ampulle (3) einen Ampullenkörper (10) aufweist, wobei der Ampullenkörper (10) zumindest bereichsweise an der Innenwand der Aufnahme (2, 102) anliegt, und
einen Abstandhalter (9, 109), der in der Aufnahme (2, 102) zwischen dem Austragskolben (7, 107) und dem Ampullenkörper (10) oder zwischen dem Förderkolben (6, 106) und dem Ampullenkörper (10) angeordnet ist, wobei der Abstandhalter (9, 109) sich in Längsrichtung der Aufnahme (2, 102) erstreckt und **dadurch gekennzeichnet, dass** der Abstandhalter (9, 109) von der Innenwand der Aufnahme (2, 102) mit einem Abstand mindestens so groß wie die Wandstärke des Ampullenkörpers (10) beabstandet ist.

2. Vorrichtung nach Anspruch 1, **dadurch gekennzeichnet, dass**
der Abstandhalter (9, 109) an der Rückseite des Austragskolbens (7, 107) oder an der Vorderseite des Förderkolbens (6, 106) befestigt ist oder dass der Abstandhalter (9, 109) um einen Ampullenkopf (11) der Ampulle (3) herum angeordnet ist, wobei der Ampullenkopf (11) einen kleineren Außendurchmesser als der Ampullenkörper (10) hat.

3. Vorrichtung nach Anspruch 1 oder 2, **dadurch gekennzeichnet, dass** an der der Ampulle (3) zugewandten Vorderseite des Förderkolbens (6, 106) und/oder an der der Ampulle (3) zugewandten Rückseite des Austragskolbens (7, 107) zumindest ein Spaltelement (12, 112) mit einer Schneidkante angeordnet ist, vorzugsweise zumindest drei oder zumindest vier Spaltelemente (12, 112) mit jeweils einer Schneidkante angeordnet sind, wobei das zumindest eine Spaltelement (12, 112) an einem zur Innenwand der Aufnahme (2, 102) liegenden Bereich der Vorderseite des Förderkolbens (6, 106) und/oder der Rückseite des Austragskolbens (7, 107) angeordnet ist, so dass das zumindest eine Spaltelement (12, 112) beim Vortreiben des Förderkolbens (6, 106) durch die Wandung des Ampullenkörpers (10) schneidet, wobei sich bevorzugt die Schneidkante radial von einer zentralen Längsachse des Förderkolbens (6, 106) und/oder des Austragskolbens (7, 107) weg erstreckt.

4. Vorrichtung nach Anspruch 3, **dadurch gekennzeichnet, dass**
die Ampulle (3) einen Ampullenkopf (11) mit einem kleineren Durchmesser als der Ampullenkörper (10) aufweist und dass
das zumindest eine Spaltelement (12, 112) und der Abstandhalter (9, 109) an der Rückseite des Austragskolbens (7, 107) angeordnet sind, wobei der Ampullenkopf (11) in Richtung des Austragskolbens (7, 107) weist, oder dass
das zumindest eine Spaltelement (12, 112) und der Abstandhalter (9, 109) an der Vorderseite des Förderkolbens (6, 106) angeordnet sind, wobei der Ampullenkopf (11) in Richtung des Förderkolbens (6, 106) weist.

5. Vorrichtung nach Anspruch 3 oder 4, **dadurch gekennzeichnet, dass**
der Abstandhalter (9, 109) in Längsrichtung wenigstens dreimal so lang ist, wie das zumindest eine Spaltelement (12, 112) in Längsrichtung von der Vorderseite des Förderkolbens (6, 106) oder der Rückseite des Austragskolbens (7, 107) absteht, wobei vorzugsweise der Abstandhalter (9, 109) in Längsrichtung wenigstens fünfmal so lang ist, wie das zumindest eine Spaltelement (12, 112) in Längsrichtung von der Vorderseite des Förderkolbens (6, 106) oder der Rückseite des Austragskolbens (7, 107) absteht.

6. Vorrichtung nach einem der vorangehenden Ansprüche, **dadurch gekennzeichnet, dass**
der Abstandhalter (9, 109) beim Vortreiben des Förderkolbens (6, 106) eine weitere Verringerung des Abstands des Förderkolbens (6, 106) zum Austragskolben (7, 107) nach dem Aufbrechen der Ampulle (3) und nach einer Komprimierung der Ampulle (3) blockiert, so dass Splitter (52) der Ampulle (3) zwischen dem Austragskolben (7, 107) und dem Förderkolben (6, 106) Platz finden ohne in kleinere Splitter (52) zerbrochen zu werden, wenn der Förderkolben (6, 106) und der Austragskolben (7, 107) mit dem Abstandhalter (9, 109) dazwischen in Richtung der Vorderseite der Kartusche (1, 101) bewegt werden.

7. Vorrichtung nach einem der vorangehenden Ansprüche, **dadurch gekennzeichnet, dass**
der Abstandhalter (9, 109) eine Länge in der Längsrichtung hat, so dass das Volumen zwischen dem Förderkolben (6, 106) und dem Austragskolben (7, 107) bei einem Abstand, der der Länge des Abstandhalters (9, 109) entspricht, größer ist als das Volumen des Materials der Ampulle (3), vorzugsweise mindestens doppelt so groß ist wie das Volumen des Materials der Ampulle (3) oder mindestens so groß ist wie das Volumen der Splitter (52) der zerbrochenen Ampulle (3) einschließlich aller Zwischenräume.

8. Vorrichtung nach einem der vorangehenden Ansprüche, **dadurch gekennzeichnet, dass**
der Abstandhalter (9, 109) durch eine Mehrzahl von Stäben (9, 109) realisiert ist, die sich in Längsrichtung erstrecken, wobei die Stäbe (9, 109) miteinander verbunden sind oder an der Vorderseite des Förderkolbens (6, 106) befestigt sind oder an der Rückseite des Austragskolbens (7, 107) befestigt sind, wobei die Stäbe (9, 109) vorzugsweise im Querschnitt rund, dreieckig, eckig oder viereckig sind und/oder in einem Längsschnitt T-förmig geformt sind.

9. Vorrichtung nach einem der vorangehenden Ansprüche, **dadurch gekennzeichnet, dass**
die Ampulle (3) einen Ampullenkopf (11) aufweist, der mit dem Ampullenkörper (10) verbunden ist, wobei der Ampullenkopf (11) einen kleineren Außendurchmesser als der Ampullenkörper (10) hat und wobei der Abstandhalter (9, 109) neben dem Ampullenkopf (11) angeordnet ist oder der Abstandhalter (9, 109) den Ampullenkopf (11) umgibt.

10. Vorrichtung nach einem der vorangehenden Ansprüche, **dadurch gekennzeichnet, dass**
die Ampulle (3) aus Glas oder aus einem gegenüber der Monomerflüssigkeit (4) chemisch stabilen Kunststoff besteht, wobei Glas als Material für die Ampulle (3) bevorzugt ist.

11. Vorrichtung nach einem der vorangehenden Ansprüche, **dadurch gekennzeichnet, dass**
der Ampullenkörper (10) zylindrisch geformt ist und die Aufnahme (2, 102) in ihrem Inneren zylindrisch geformt ist, wobei vorzugsweise der Außendurchmesser des Ampullenkörpers (10) zum Innendurchmesser der im Inneren zylindrisch geformten Aufnahme (2, 102) passt, so dass der Ampullenkörper (10) in der Aufnahme (2, 102) gehalten ist.

12. Vorrichtung nach einem der vorangehenden Ansprüche, **dadurch gekennzeichnet, dass**
der Förderkolben (6, 106) von einer Rückseite der Aufnahme (2, 102) aus in der Längsrichtung zur Vorderseite vortreibbar gelagert ist.

13. Vorrichtung nach einem der vorangehenden Ansprüche, **dadurch gekennzeichnet, dass**
die Rückseite der Kartusche (1, 101) mit der Vorderseite der Aufnahme (2, 102) derart verbunden ist, dass der Innenraum der Kartusche (1, 101) mit dem Innenraum der Aufnahme (2, 102) fluchtet.

14. Vorrichtung nach einem der vorangehenden Ansprüche, **dadurch gekennzeichnet, dass**
das Zementpulver (5) im Innenraum der Kartusche (1, 101) zwischen der Vorderseite der Kartusche (1, 101) und dem Austragskolben (7, 107) angeordnet ist, wobei vorzugsweise in dem Zementpulver (5) ein die Monomerflüssigkeit (4) leitendes Additiv verteilt ist.

15. Vorrichtung nach einem der vorangehenden Ansprüche, **dadurch gekennzeichnet, dass**
an der Vorderseite des Austragskolbens (7, 107) ein Hohlzylinder (15, 115) angeordnet ist, der eine weitere Bewegung des Austragskolbens (7, 107) in Richtung der Vorderseite der Kartusche (1, 101) blockiert, so dass der Austragskolben (7, 107) bereichsweise von der Vorderseite des Innenraums der Kartusche (1, 101) beabstandet ist und ein Totvolumen in dem Innenraum der Kartusche (1, 101) verbleibt, wenn der Austragskolben (7, 107) gegen die Vorderseite des Innenraums der Kartusche (1, 101) gedrückt ist.

16. Verfahren zur Herstellung eines Knochenzementteigs (54), insbesondere eines pastenförmigen Polymethylmethacrylat-Knochenzementteigs (54), wobei der Knochenzementteig (54) aus einem Zementpulver (5) und einer Monomerflüssigkeit (4) hergestellt wird, **gekennzeichnet durch** die folgenden Schritte:
A) zwischen einem in einer Längsrichtung beweglichen Förderkolben (6, 106) und einem Austragskolben (7, 107) sind eine Ampulle (3) enthaltend die Monomerflüssigkeit (4) und ein sich in einer Längsrichtung erstreckender Abstandhalter (9, 109) in einer Aufnahme (2, 102) angeordnet, wobei der Förderkolben (6, 106) in Längsrichtung zum Austragskolben (7, 107) hin gedrückt wird,
B) durch die Bewegung des Förderkolbens (6, 106) gegen den Austragskolben (7, 107) wird ein Ampullenkopf (11) der Ampulle (3) aufgebrochen oder abgebrochen, **dadurch gekennzeichnet, dass** ein freies Ende des Abstandhalters (9, 109) im Inneren der geöffneten Ampulle (3) gegen einen Ampullenkörper (10) der geöffneten Ampulle (3) bewegt wird, so dass während der Bewegung zumindest ein Teil der Wandung des Ampullenkörpers (10) zwischen dem Abstandhalter (9, 109) und einer Innenwand der Aufnahme (2, 102) angeordnet ist,
C) die geöffnete Ampulle (3) wird durch die Bewegung des Förderkolbens (6, 106) gegen den Austragskolben (7, 107) komprimiert und weiter zerbrochen und die Monomerflüssigkeit (4) wird dabei aus der Aufnahme (2, 102) heraus und in das Zementpulver (5) hineingepresst, wo sie sich mit dem Zementpulver (5) unter Bildung des Knochenzementteigs (54) vermischt,
D) der Abstandhalter (9, 109) wird zwischen dem Förderkolben (6, 106) und dem Austragskolben (7, 107) eingeklemmt und verhindert dadurch eine weitere Reduzierung des Abstands des Austragskolbens (7, 107) zum Förderkolben (6, 106) und damit ein weiteres Auspressen von Monomerflüssigkeit (4) aus der Aufnahme (2, 102) in den Knochenzementteig.

17. Verfahren nach Anspruch 16, **dadurch gekennzeichnet, dass**
das Verfahren mit einer Vorrichtung nach einem der Ansprüche 1 bis 15 durchgeführt wird.

18. Verfahren nach Anspruch 16 oder 17, **dadurch gekennzeichnet, dass** in Schritt C) die Monomerflüssigkeit (4) durch zumindest eine für das Zementpulver (5) undurchlässige aber für Gase und die Monomerflüssigkeit (4) durchlässige Verbindung im Austragskolben (7, 107) in eine Kartusche (1, 101) enthaltend das Zementpulver (5) hineingepresst wird.

19. Verfahren nach einem der Ansprüche 16 bis 18, **dadurch gekennzeichnet, dass** die Bewegung des Förderkolbens (6, 106) in Schritt B) und C) durch eine axiale Bewegung einer Stange (44) einer Auspressvorrichtung (43) angetrieben wird, die vor Schritt A) an der Aufnahme (2, 102) befestigt wird.

20. Verfahren nach einem der Ansprüche 16 bis 19, **dadurch gekennzeichnet, dass** an der Rückseite des Austragskolbens (7, 107) oder an der Vorderseite des Förderkolbens (6, 106) zumindest ein Spaltelement (12, 112) mit einer Schneidkante angeordnet ist, wobei in Schritt C) die Wandung des Ampullenkörpers (10) mit der Schneidkante geschnitten oder gebrochen wird, wobei das freie Ende des Abstandhalters (9, 109) von dem zumindest einen Spaltelement (12, 112) beabstandet ist, vorzugsweise mit einem Abstand in Längsrichtung von mindestens 10 mm beabstandet ist.

21. Verfahren nach einem der Ansprüche 16 bis 20, **dadurch gekennzeichnet, dass** das Zementpulver (5) in einem Innenraum einer Kartusche (1, 101) angeordnet ist, wobei der Austragskolben (7, 107) in Längsrichtung beweglich in dem Innenraum der Kartusche (1, 101) angeordnet ist, wobei in Schritt C) die Monomerflüssigkeit (4) in den Innenraum der Kartusche (1, 101) gepresst wird und wobei nach Schritt D) in einem Schritt E) der Austragskolben (7, 107) von dem Förderkolben (6, 106) in Längsrichtung in den Innenraum der Kartusche (1, 101) gedrückt wird und dabei der Knochenzementteig (54) aus dem Innenraum der Kartusche (1, 101) herausgedrückt wird.

## Claims

1. A device for producing a bone cement paste (54) from a monomer liquid (4) and a cement powder (5) as starting components of the bone cement paste (54) and for discharging the mixed bone cement paste (54), the device having:
a cartridge (1, 101) comprising a cylindrical interior, wherein a discharging plunger (7, 107), which can move towards a front side of the cartridge (1, 101), is arranged in the interior of the cartridge (1, 101) in a rear side of the cartridge (1, 101),
a receptacle (2, 102), which extends along a longitudinal direction, wherein a front side of the receptacle (2, 102) is connected to the rear side of the cartridge (1, 101),
a delivery plunger (6, 106), which is arranged in the receptacle (2, 102), wherein the delivery plunger (6, 106) is supported in the receptacle (2, 102) so that it can move in the longitudinal direction of the receptacle (2, 102) towards the front side of the receptacle (2, 102),
a breakable ampoule (3) containing the monomer liquid (4), wherein the ampoule (3) is arranged in the receptacle (2, 102) between the delivery plunger (6, 106) and the discharging plunger (7, 107), and wherein the ampoule (3) has an ampoule body (10), wherein the ampoule body (10) at least partially abuts on the inner wall of the receptacle (2, 102), and
a spacer (9, 109), which is arranged in the receptacle (2, 102) between the discharging plunger (7, 107) and the ampoule body (10) or between the delivery plunger (6, 106) and the ampoule body (10), wherein the spacer (9, 109) extends in the longitudinal direction of the receptacle (2, 102) and **characterised in that**
the spacer (9, 109) is spaced apart from the inner wall of the receptacle (2, 102) at a distance, which is at least as large as the wall thickness of the ampoule body (10).

2. The device according to claim 1, **characterised in that**
the spacer (9, 109) is fastened to the rear side of the discharging plunger (7, 107) or to the front side of the delivery plunger (6, 106) or that the spacer (9, 109) is arranged around an ampoule head (11) of the ampoule (3), wherein the ampoule head (11) has a smaller outer diameter than the ampoule body (10).

3. The device according to claim 1 or 2, **characterised in that** at least one splitting element (12, 112) comprising a cutting edge, preferably at least three or at least four splitting elements (12, 112), each comprising a cutting edge, is arranged/are arranged on the front side of the delivery plunger (6, 106) facing the ampoule (3) and/or on the rear side of the discharging plunger (7, 107) facing the ampoule (3), wherein the at least one splitting element (12, 112) is arranged at an area located towards the inner wall of the receptacle (2, 102) of the front side of the delivery plunger (6, 106) and/or of the rear side of the discharging plunger (7, 107), so that the at least one splitting element (12, 112) cuts through the wall of the ampoule body (10) when the delivery plunger (6, 106) is advanced, wherein the cutting edge preferably extends radially away from a central longitudinal axis of the delivery plunger (6, 106) and/or of the discharging plunger (7, 107).

4. The device according to claim 3, **characterised in that**
the ampoule (3) has an ampoule head (11) with a smaller diameter than the ampoule body (10), and that
the at least one splitting element (12, 112) and the spacer (9, 109) are arranged on the rear side of the discharging plunger (7, 107), wherein the ampoule head (11) points towards the discharging plunger (7, 107), or that the at least one splitting element (12, 112) and the spacer (9, 109) are arranged on the front side of the delivery plunger (6, 106), wherein the ampoule head (11) points in the direction of the delivery plunger (6, 106).

5. The device according to claim 3 or 4, **characterised in that** in the longitudinal direction, the spacer (9, 109) is at least three times as long as the at least one splitting element (12, 112) sticks out from the front side of the delivery plunger (6, 106) or from the rear side of the discharging plunger (7, 107) in the longitudinal direction, wherein in the longitudinal direction, the spacer (9, 109) is preferably at least five times as long as the at least one splitting element (12, 112) sticks out from the front side of the delivery plunger (6, 106) or from the rear side of the discharging plunger (7, 107) in the longitudinal direction.

6. The device according to any one of the preceding claims, **characterised in that** when advancing the delivery plunger (6, 106), the spacer (9, 109) blocks a further decrease of the distance between the delivery plunger (6, 106) and the discharging plunger (7, 107) after the ampoule (3) has been broken open and after the ampoule (3) has been compressed, so that shards (52) of the ampoule (3) can be accommodated between the discharging plunger (7, 107) and the delivery plunger (6, 106), without being broken into smaller shards (52) when the delivery plunger (6, 106) and the discharging plunger (7, 107) with the spacer (9, 109) in-between are moved towards the front side of the cartridge (1, 101).

7. The device according to any one of the preceding claims, **characterised in that** the spacer (9, 109) has a length in the longitudinal direction so that the volume between the delivery plunger (6, 106) and the discharging plunger (7, 107) at a distance, which corresponds to the length of the spacer (9, 109), is larger than the volume of the material of the ampoule (3), preferably at least twice as large as the volume of the material of the ampoule (3), or at least as large as the volume of the chards (52) of the broken ampoule (3) including all spaces.

8. The device according to any one of the preceding claims, **characterised in that** the spacer (9, 109) is realised by a plurality of rods (9, 109), which extend in the longitudinal direction, wherein the rods (9, 109) are connected to one another or are fastened to the front side of the delivery plunger (6, 106) or are fastened to the rear side of the discharging plunger (7, 107), wherein the rods (9, 109) preferably have a round, triangular, angular or square cross section and/or are formed in a T-shaped manner in a longitudinal section.

9. The device according to any one of the preceding claims, **characterised in that** the ampoule (3) has an ampoule head (11), which is connected to the ampoule body (10), wherein the ampoule head (11) has a smaller outer diameter than the ampoule body (10), and wherein the spacer (9, 109) is arranged next to the ampoule head (11) or the spacer (9, 109) surrounds the ampoule head (11).

10. The device according to any one of the preceding claims, **characterised in that** the ampoule (3) consists of glass or of a plastic, which is chemically stable against the monomer liquid (4), wherein glass is preferred as material for the ampoule (3).

11. The device according to any one of the preceding claims, **characterised in that** the ampoule body (10) is formed cylindrically and the receptacle (2, 102) is formed cylindrically in its interior, wherein the outer diameter of the ampoule body (10) preferably matches the inner diameter of the receptacle (2, 102), which is formed cylindrically in the interior, so that the ampoule body (10) is held in the receptacle (2, 102).

12. The device according to any one of the preceding claims, **characterised in that** the delivery plunger (6, 106) is supported so that it can be advanced from a rear side of the receptacle (2, 102) to the front side in the longitudinal direction.

13. The device according to any one of the preceding claims, **characterised in that** the rear side of the cartridge (1, 101) is connected to the front side of the receptacle (2, 102) such that the interior of the cartridge (1, 101) is aligned with the interior of the receptacle (2, 102).

14. The device according to any one of the preceding claims, **characterised in that** the cement powder (5) is arranged in the interior of the cartridge (1, 101) between the front side of the cartridge (1, 101) and the discharging piston (7, 107), wherein an additive, which conducts the monomer liquid (4), is preferably distributed in the cement powder (5).

15. The device according to any one of the preceding claims, **characterised in that** a hollow cylinder (15, 115), which blocks a further movement of the discharging plunger (7, 107) towards the front side of the cartridge (1, 101), is arranged on the front side of the discharging plunger (7, 107), so that the discharging plunger (7, 107) is partially spaced apart from the front side of the interior of the cartridge (1, 101), and a dead volume remains in the interior of the cartridge (1, 101) when the discharging plunger (7, 107) is pushed against the front side of the interior of the cartridge (1, 101).

16. A method for producing a bone cement paste (54), in particular a pasty polymethyl methacrylate bone cement paste (54), wherein the bone cement paste (54) is produced from a cement powder (5) and a monomer liquid (4), **characterised by** the following steps:
A) an ampoule (3) containing the monomer liquid (4) and a spacer (9, 109) extending in a longitudinal direction are arranged in a receptacle (2, 102) between a delivery plunger (6, 106), which can move in a longitudinal direction, and a discharging plunger (7, 107), wherein the delivery plunger (6, 106) is pushed towards the discharging plunger (7, 107) in the longitudinal direction,
B) an ampoule head (11) of the ampoule (3) is broken open or broken off by the movement of the delivery plunger (6, 106) against the discharging plunger (7, 107), **characterised in that** a free end of the spacer (9, 109) in the interior of the open ampoule (3) is moved against an ampoule body (10) of the open ampoule (3), so that at least a part of the wall of the ampoule body (10) is arranged between the spacer (9, 109) and an inner wall of the receptacle (2, 102) during the movement,
C) the open ampoule (3) is compressed and further broken by the movement of the delivery plunger (6, 106) towards the discharging plunger (7, 107), and the monomer liquid (4) is thereby squeezed out of the receptacle (2, 102) and into the cement powder (5), where it mixes with the cement powder (5) by forming the bone cement paste (54),
D) the spacer (9, 109) is clamped between the delivery plunger (6, 106) and the discharging plunger (7, 107) and thus prevents a further reduction of the distance between the discharging plunger (7, 107) and the delivery plunger (6, 106), and thus a further squeezing of monomer liquid (4) out of the receptacle (2, 102) into the bone cement paste.

17. The method according to claim 16, **characterised in that** the method is performed with a device according to any one of claims 1 to 15.

18. The method according to claim 16 or 17, **characterised in that** in step C), the monomer liquid (4) is squeezed through at least one connection in the discharging plunger (7, 107), which is impermeable for the cement powder (5) but permeable for gases and the monomer liquid (4), into a cartridge (1, 101) containing the cement powder (5).

19. The method according to any one of claims 16 to 18, **characterised in that** the movement of the delivery plunger (6, 106) in step B) and C) is driven by an axial movement of a rod (44) of a squeezing device (43), which is fastened to the receptacle (2, 102) prior to step A).

20. The method according to any one of claims 16 to 19, **characterised in that** at least one splitting element (12, 112) comprising a cutting edge is arranged on the rear side of the discharging plunger (7, 107) or on the front side of the delivery plunger (6, 106), wherein the wall of the ampoule body (10) is cut or broken by means of the cutting edge in step C), wherein the free end of the spacer (9, 109) is spaced apart from the at least one splitting element (12, 112), preferably spaced apart at a distance in the longitudinal direction of at least 10 mm.

21. The method according to any one of claims 16 to 20, **characterised in that** the cement powder (5) is arranged in an interior of a cartridge (1, 101), wherein the discharging plunger (7, 107) is arranged in the interior of the cartridge (1, 101) so that it can move in the longitudinal direction, wherein the monomer liquid (4) is pushed into the interior of the cartridge (1, 101) in step C), and wherein after step D) in a step E) the discharging plunger (7, 107) is pushed by the delivery plunger (6, 106) in the longitudinal direction into the interior of the cartridge (1, 101), and the bone cement paste (54) is thereby pushed out of the interior of the cartridge (1, 101)

## Revendications

1. Dispositif pour la fabrication d'une pâte de ciment osseux (54) à partir d'un liquide monomère (4) et d'une poudre de ciment (5) comme composants de départ de la pâte de ciment osseux (54) et pour le déversement de la pâte de ciment osseux (54) mélangée, le dispositif présentant
une cartouche (1, 101) avec un espace intérieur cylindrique, un piston de décharge (7, 107) dans une face arrière de la cartouche (1, 101), mobile en direction d'une face frontale de la cartouche (1, 101), étant disposé dans l'espace intérieur de la cartouche (1, 101),
un logement (2, 102) qui s'étend dans un sens longitudinal, une face frontale du logement (2, 102) étant reliée à la face arrière de la cartouche (1, 101),
un piston d'alimentation (6, 106) qui est disposé dans le logement (2, 102), le piston d'alimentation (6, 106) étant logé de façon mobile dans le logement (2, 102) dans le sens longitudinal du logement (2, 102) vers la face frontale du logement (2, 102),
une ampoule (3) contenant le liquide monomère (4) pouvant être rompue, l'ampoule (3) étant disposée entre le piston d'alimentation (6, 106) et le piston de décharge (7, 707) dans le logement (2, 102) et l'ampoule (3) présentant un corps d'ampoule (10), le corps d'ampoule (10) étant adjacent au moins partiellement avec la paroi intérieure du logement (2, 102) et
une entretoise (9, 109) qui est disposée dans le logement (2, 102) entre le piston de décharge (7, 107) et le corps d'ampoule (10) ou entre le piston d'alimentation (7, 107) et le corps d'ampoule (10), l'entretoise (9, 109) s'étendant dans le sens longitudinal du logement (2, 102) et
**caractérisé en ce que**
l'entretoise (9, 109) est espacée de la paroi intérieure du logement (2, 102) à une distance au moins aussi grande que l'épaisseur de paroi du corps d'ampoule (10).

2. Dispositif conformément à la revendication n°1, **caractérisé en ce que** l'entretoise (9, 109) est fixée sur la face arrière du piston de décharge (7, 107) ou sur la face frontale du piston d'alimentation (6, 106) ou que l'entretoise (9, 109) est disposée autour d'une tête d'ampoule (11) de l'ampoule (3), la tête d'ampoule (11) ayant un diamètre extérieur plus petit que le corps d'ampoule (10).

3. Dispositif conformément à la revendication n°1 ou n°2, **caractérisé en ce que** au moins un élément de scission (12, 112) avec un bord coupant, préférablement au moins trois ou au moins quatre éléments de scission (12, 112) comportant chacun un bord coupant étant disposés sur la face frontale du piston d'alimentation (6, 106), tournée vers l'ampoule (3), et/ou sur la face arrière du piston de décharge (7, 107), tournée vers l'ampoule (3), au moins un élément de scission (12, 112) étant disposé dans une zone située vers la paroi intérieure du logement (2, 102) de la face frontale du piston d'alimentation (6, 106) et/ou de la face arrière du piston de décharge (7, 107) de façon à ce qu'au moins un élément de scission (12, 112) coupe la paroi du corps d'ampoule (10) lors de l'avance du piston d'alimentation (6, 106), le bord coupant s'étendant préférablement dans le sens radial depuis un axe longitudinal central du piston d'alimentation (6, 106) et/ou du piston de décharge (7, 107).

4. Dispositif conformément à la revendication n°3, **caractérisé en ce que** l'ampoule (3) présente une tête d'ampoule (11) avec un plus petit diamètre que le corps d'ampoule (10) et **en ce que**
au moins un élément de scission (12, 112) et l'entretoise (9, 109) sont disposés sur la face arrière du piston de décharge (7, 107), la tête d'ampoule (11) étant tournée vers le piston de décharge (7, 107) ou **en ce que** au moins un élément de scission (12, 112) et l'entretoise (9, 109) sont disposés sur la face frontale du piston d'alimentation (6, 106), la tête d'ampoule (11) étant dirigée vers le piston d'alimentation (6, 106).

5. Dispositif conformément à la revendication n°3 ou n°4, **caractérisé en ce que** l'entretoise (9, 109) est, dans le sens longitudinal, au moins trois fois plus longue que la distance entre au moins un élément de scission (12, 112) dans le sens longitudinal et la face frontale du piston d'alimentation (6, 106) ou la face arrière du piston de décharge (7, 107), l'entretoise (9, 109) étant, dans le sens longitudinal, préférablement au moins cinq fois plus longue que la distance entre au moins un élément de scission (12, 112) dans le sens longitudinal et la face frontale du piston d'alimentation (6, 106) ou la face arrière du piston de décharge (7, 107).

6. Dispositif conformément à l'une des revendications précédentes, **caractérisé en ce que**
l'entretoise (9, 109) bloque une autre diminution de la distance entre le piston d'alimentation (6, 106) et le piston de décharge (7, 107) après la rupture de l'ampoule (3) et après une compression de l'ampoule (3) lors de l'avance du piston d'alimentation (6, 106) de façon à ce que des éclats (52) de l'ampoule (3) se trouvent entre le piston de décharge (7, 107) et le piston d'alimentation (6, 106) sans être cassés en plus petits fragments (52) lorsque le piston d'alimentation (6, 106) et le piston de décharge (7, 107) sont déplacés, avec l'entretoise (9, 109) entre ces derniers, vers la face frontale de la cartouche (1, 101).

7. Dispositif conformément à l'une des revendications précédentes, **caractérisé en ce que**
l'entretoise (9, 109) a, dans le sens longitudinal, une longueur telle que le volume entre le piston d'alimentation (6, 106) et le piston de décharge (7, 107), à une distance qui correspond à la longueur de l'entretoise (9, 109), est plus grand que le volume du matériau de l'ampoule (3), préférablement au moins deux fois plus grand que le volume du matériau de l'ampoule (3) ou au moins aussi grand que le volume des éclats (52) de l'ampoule (3) cassée, tous les interstices étant inclus.

8. Dispositif conformément à l'une des revendications précédentes, **caractérisé en ce que**
l'entretoise (9, 109) est réalisée par une pluralité de montants (9, 109) qui s'étendent dans le sens longitudinal, les montants (9, 109) étant reliés entre eux ou fixés sur la face frontale du piston d'alimentation (6, 106) ou sur la face arrière du piston de décharge (7, 107), les montants (9, 109) étant préférablement de section ronde, triangulaire, angulaire ou carrée et/ou en forme de T dans une coupe longitudinale.

9. Dispositif conformément à l'une des revendications précédentes, **caractérisé en ce que**
l'ampoule (3) présente une tête d'ampoule (11) qui est reliée au corps d'ampoule (10), la tête d'ampoule (11) présentant un plus petit diamètre extérieur que le corps d'ampoule (10) et l'entretoise (9, 109) étant disposée à côté de la tête d'ampoule (11) ou l'entretoise (9, 109) entourant la tête d'ampoule (11).

10. Dispositif conformément à l'une des revendications précédentes, **caractérisé en ce que**
l'ampoule (3) se compose de verre ou d'une matière plastique chimiquement stable par rapport au liquide monomère (4), le verre étant préférentiellement le matériau d'ampoule (3).

11. Dispositif conformément à l'une des revendications précédentes, **caractérisé en ce que**
le corps d'ampoule (10) est de forme cylindrique et le logement (2, 102) est de forme cylindrique à l'intérieur, le diamètre extérieur du corps d'ampoule (10) s'adaptant préférablement au diamètre intérieur du logement (2, 102) de forme cylindrique à l'intérieur de façon à ce que le corps d'ampoule (10) soit maintenu dans le logement (2, 102).

12. Dispositif conformément à l'une des revendications précédentes, **caractérisé en ce que**
le piston d'alimentation (6, 106) est logé de manière à pouvoir avancer depuis une face arrière du logement (2, 102) dans le sens longitudinal vers la face frontale.

13. Dispositif conformément à l'une des revendications précédentes, **caractérisé en ce que**
la face arrière de la cartouche (1, 101) est reliée à la face frontale du logement (2, 102) de telle manière à ce que l'espace intérieur de la cartouche (1, 101) soit aligné avec l'espace intérieur du logement (2, 102).

14. Dispositif conformément à l'une des revendications précédentes, **caractérisé en ce que**
la poudre de ciment (5) dans l'espace intérieur de la cartouche (1, 101) est disposée entre la face frontale de la cartouche (1, 101) et le piston de décharge (7,107), un additif conduisant le liquide monomère (4) étant préférablement réparti dans la poudre de ciment (5).

15. Dispositif conformément à l'une des revendications précédentes, **caractérisé en ce que**,
sur la face frontale du piston de décharge (7,107) est disposé un cylindre creux (15, 115) qui bloque un autre mouvement du piston de décharge (7,107) vers la face frontale de la cartouche (1, 101) de façon à ce que le piston de décharge (7,107) soit partiellement espacé de la face frontale de l'espace intérieur de la cartouche (1, 101) et qu'un volume mort reste dans l'espace intérieur de la cartouche (1, 101) lorsque le piston de décharge (7,107) est poussé contre la face frontale de l'espace intérieur de la cartouche (1, 101).

16. Procédé pour la fabrication d'une pâte de ciment osseux (54), en particulier d'une pâte de ciment osseux (54) de méthacrylate de polyméthyle sous forme pâteuse, la pâte de ciment osseux (54) étant fabriquée à partir d'une poudre de ciment (5) et d'un liquide monomère (4), **caractérisé par** les étapes suivantes :
A) Une ampoule (3) contenant le liquide monomère (4) et une entretoise (9, 109) s'étendant dans un sens longitudinal dans un logement (2, 102) sont disposées entre un piston d'alimentation (6, 106) mobile dans un sens longitudinal et un piston de décharge (7, 107), le piston d'alimentation (6, 106) étant pressé vers le piston de décharge (6, 106) dans le sens longitudinal.
B) Une tête d'ampoule (11) de l'ampoule (3) est cassée ou rompue par le mouvement du piston d'alimentation (6, 106) contre le piston de décharge (7, 107),
**caractérisée en ce que**
l'extrémité libre de l'entretoise (9, 109) à l'intérieur de l'ampoule (3) ouverte est déplacée contre un corps d'ampoule (10) de l'ampoule (3) ouverte de façon à ce que, pendant le mouvement, au moins une partie de la paroi du corps d'ampoule (10) soit disposée entre l'entretoise (9, 109) et une paroi intérieure du logement (2, 102).
C) L'ampoule (3) ouverte est comprimée par le mouvement du piston d'alimentation (6, 106) contre le piston de décharge (7, 107) et davantage cassée, le liquide monomère (4) s'échappe ainsi du logement (2, 102) et est pressé dans la poudre de ciment (4) où il se mélange avec la poudre de ciment (4) en formant la pâte de ciment osseux (54).
D) L'entretoise (9, 109) est coincée entre le piston d'alimentation (6, 106) et le piston de décharge (7, 107) et empêche ainsi une diminution supplémentaire de la distance entre le piston de décharge (7, 107) et le piston d'alimentation (6, 106) et, par conséquent, une extraction supplémentaire du liquide monomère (4) du logement (2, 102) dans la pâte de ciment osseux.

17. Procédé conformément à la revendication n°16, **caractérisé en ce que** le procédé est réalisé avec un dispositif conformément à l'une des revendications n°1 à n°15.

18. Procédé conformément à la revendication n°16 ou n°17, **caractérisé en ce que** dans l'étape C), le liquide monomère (4) est pressé dans une cartouche (1, 101) contenant la poudre de ciment (5) par au moins un composé imperméable à la poudre de ciment (5) mais perméable aux gaz et au liquide monomère (4) dans le piston de décharge (7,107).

19. Procédé conformément à l'une des revendications n°16 à n°18, **caractérisé en ce que**
le mouvement du piston d'alimentation (6, 106) dans les étapes B) et C) est entraîné par un mouvement axial de la tige (44) d'un dispositif d'extraction (43) qui est fixé sur le logement (2, 102) avant l'étape A).

20. Procédé conformément à l'une des revendications n°16 à n°19, **caractérisé en ce que**
au moins un élément de scission (12, 112) avec un bord coupant est disposé sur la face arrière du piston de décharge (7, 707) ou sur la face frontale du piston d'alimentation (6, 606), la paroi du corps d'ampoule (10) avec le bord coupant étant coupée ou rompue dans l'étape C), l'extrémité libre de l'entretoise (9, 109) étant espacée au moins d'un élément de scission (12, 112), préférablement à une distance dans le sens longitudinal d'au moins 10 mm.

21. Procédé conformément à l'une des revendications n°16 à n°20, **caractérisé en ce que**,
la poudre de ciment (5) est disposée dans un espace intérieur d'une cartouche (1, 101), le piston de décharge (7,107) étant disposé en étant mobile dans le sens longitudinal dans l'espace intérieur de la cartouche (1, 101), le liquide monomère (4) étant pressé dans l'espace intérieur de la cartouche (1, 101) dans l'étape C) et le piston de décharge (7, 707) étant pressé par le piston d'alimentation (6, 606) dans le sens longitudinal dans l'espace intérieur de la cartouche (1, 101) dans une étape E) après l'étape D) et la pâte de ciment osseux (54) étant ainsi extraite de l'espace intérieur de la cartouche (1, 101).
